# EUROPEAN PATENT APPLICATION

(11) **EP 0 061 745 A1**
(43) Date of publication of application: **06.10.1982**
(21) Application number: 82102569.9
(22) Date of filing: 26.03.1982
(51) Int. Cl.: C07D 217/26, A61K 31/47

(54) **Tetrahydroisoquinoline compounds, processes for preparation thereof and pharmaceutical composition containing them**

(30) Priority: 27.03.1981 JP 45727/81
(71) Applicant: Tanabe Seiyaku Co., Ltd., Osaka-shi Osaka-fu (JP)
(72) Inventor: Yoneda, Naoto, Suita-shi Osaka-fu (JP); Kato, Jyoji, Yawata-shi Kyoto-fu (JP); Kinashi, Keizo, Yao-shi Osaka-fu (JP); Hayashi, Kimiaki, Suita-shi Osaka-fu (JP)
(74) Representative: Hansen, Bernd, Dr. Dipl.-Chem.

(57) **Abstract**

Novel tetrahydroisoquinoline compounds of the formula: 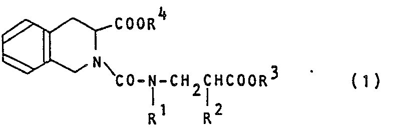 wherein R¹ is lower alkyl or phenyl-lower alkyl, R² is alkyl or phenyl-lower alkyl, R³ is hydrogen or lower alkyl and R⁴ is hydrogen, lower alkyl or aralkyl, and pharmaceutically acceptable salts thereof are disclosed. Said compounds (I) and salts thereof are useful as hypotensive agent.

There is also described a process for preparation of said compounds as well as pharmaceutical compositions containing same.

## Description

This invention relates to novel tetrahydroisoquinoline compounds, processes for preparation thereof and pharmaceutical compositions containing them. More particularly it relates to a compound of the formula: 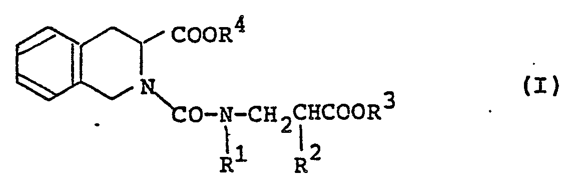 wherein _{R}ⁱ is lower alkyl or phenyl-lower alkyl, R² is alkyl or phenyl-lower alkyl, _{R}³ is hydrogen or lower alkyl and _{R}⁴ is hydrogen, lower alkyl or aralkyl, or a pharmaceutically acceptable salt thereof.

Angiotensin I is converted by angiotensin-converting enzyme (ACE) to antiotensin II. Said antiogensin II induces strong constriction of vascular smooth muscle and is causative of various forms of hypertension in mammalian species. On the other hand, endogenous prostacyclin (PG I₂), which is derived from arachidonic acid via prostaglandin H₂ (PG H₂) by the action of PG I₂-synthetase, is a potent hypotensive substance and causes a fall in blood pressure of, for example, spontaneously hypertensive rats (c.f., Advances in Prostaglandin and Thromboxane Research Vol. 7 (1980), pages 815 - 819).

As a result of investigations we have now found that, the tetrahydroisoquinoline compound (I) or a salt thereof shows potent ACE-inhibitory effect and at the same time stimulates the production of PG I₂, a vasodepressor substance, and is useful as a blood-pressure lowering agent. For example, when said inhibitory effect is estimated in vitro by the use of ACE isolated from pig's kidney, (3S)-2-[N-((2R)-2-carboxy-n-decyl)-N-ethylcarbamoylJ-1,2,3,4-tetrahydroisoquinoline-3-carboxylic acid at a concentration of 4.2 x 10⁹ mol/liter shows 50 % inhibition of the enzymatic activity of ACE. On the other hand, when administered orally to spontaneously hypertensive rats (SHR), (3S)-2-[N-((2R)-2- ethoxycarbonyl-4-phenyl-n-butyl)-N-ethylcarbamoyl)-1,2,3,4-tetrahydroisoquinoline-3-carboxylic acid 1/2 calcium salt or (3S)-2-[N-((2R)-2-ethoxycarbonyl-n-decyl)-N-ethylcarbamoyl]-1,2,3,4-tetrahydroisoquinoline-3-carboxylic acid 1/2 calcium salt at a dose of 50.mg/kg shows a decrease of about 35 mmHg in the blood pressure of said SHR and this blood-pressure lowering effect of the compound lasts for more than 6 hours. Moreover, when the effect of a test compound on the production of PG 1₂ in vascular tissues is estimated by the use of pig's blood platelet lysate or microsome obtained from pig's aorta, (3S)-2-[N-((2R)-2-carboxy-n-dodecyl)-N-ethylcarbamoyl]-1,2,3,4-tetrahydroisoquinoline-3-carboxylic acid shows 62 _{%} increase in the formation of PG I₂ as compared with a control group. In addition, the compound (I) of the invention . shows no substantial decrease in its hypotensive activity (i.e., no substantial tolerance) even after the repeated administration thereof to mammlian species. For example, when (3S)-2-[N-((2R)-2-ethoxycarbonyl-n-decyl)-N-ethylcarbamoyl]-1,2,3,4-tetrahydroisoquinoline-3-cacrboxylic acid 1/2 calcium salt is administered to SHR for 15 consequtive days, said compound can maintain almost the same potency of its hypotensive activity during the experiments. The compound (I) is further characteristic in that, in using said compound as the hypotensive agent, it can also inhibit the aggregation of blood platelet because it inhibits the biosynthesis of thromboxane _{A2}, i.e., a potent vasoconstrictor which induces blood platelet aggregation.

In the compound (I) of the present invention, representative examples of R¹ include alkyl such as methyl, ethyl, propyl, butyl or pentyl; and phenyl-lower alkyl such as benzyl, phenethyl, phenylpropyl or phenylbutyl. On the other hand, representative examples of R² include alkyl such as methyl, ethyl, propyl, butyl, pentyl, hexyl, heptyl, octyl, nonyl, decyl, undecyl, dodecyl, tridecyl, tetradecyl, pentadecyl or hexadecyl; and phenyl-lower alkyl such as benzyl, phenethyl, phenylpropyl or phenylbutyl. _{R}epresen- tative examples of R³ include hydrogen and lower alkyl such as methyl, ethyl, propyl or butyl. Representative examples of R⁴ are hydrogen; lower alkyl such as methyl, ethyl, propyl or butyl; and aralkyl such as benzyl, phenethyl, p-methoxybenzyl, dimethylphenyl or trimethyl phenyl. Among the compounds of the present invention, a preferred subgenus is those of the formula (I) in which R¹ is alkyl of one to 3 carbon atoms, benzyl or phenetyl, R² is alkyl of one to 16 carbon atoms, benzyl, phenethyl or phenylpropyl and _{R}³ is hydrogen, alkyl of one to 2 carbon atoms, benzyl or phenethyl. More preferred subgenus is the compound of the formula (I) in which _{R}¹ is ethyl, R² is alkyl of 6 to 12 carbon atoms or phenethyl, R³ is ethyl and R⁴ is hydrogen.

While the compound (I) of the present invention can exist in the form of two diastereoisomers or four optical isomers due to the two asymmetric carbon atoms involved therein, all of these optical isomers or a mixture thereof are included within the scope of the invention. Among said optical isomers, however, the compound (I) in which the carbon atom at the 3rd-position of isoquinoline ring has S-configuration, especially the one in which the carbon atom at the 3rd-position of isoquinoline ring has S-configuration and the carbon atom at the 2nd-position of the propionic moiety of the formula: -CH₂-CH(R²)-COOR³ has R-configuration, is preferred for medicinal use.

The invention also includes pharmaceutical compositions comprising compounds according to formula (I) or a pharmaceutically acceptable salt thereof in a therapeutically effective amount together with pharmaceuticalla acceptable carrierse.

The compound (I) of the invention can be used for pharmaceutical use either as the free acid or a salt thereof. Pharmaceutically acceptable salts of the compound (I) include, for example, inorganic salts such as sodium, potassium, calcium and magnesium salts, organic salts such as lysine and arginine salts, and the like. , A daily dose of the compound (I) or a salt thereof may be about 30 mg to about 3 g, especially 50 mg to one g, per body of patients. Further, the compound (I) or a salt thereof may be used in the form of a pharmaceutical preparation containing the same compound in conjunction or admixture with a pharmaceutical excipient suitable for oral or parenteral administration. Suitable excipients include, for example, starch, lactose, glucose, potassium phosphate, corn starch, arabic gum, stearic acid and other known medicinal excipients. The pharmaceutical preparations may be in solid form such as tablets, pills, capsules or suppositories; or in liquid form such as solutions, suspensions or emulsions. They may be sterilized and/or may further contain auxiliaries such as stabilizing, wetting or emulsigying agent.

The compound (I) of the present invention can be prepared according to the methods shown by the following reaction scheme: 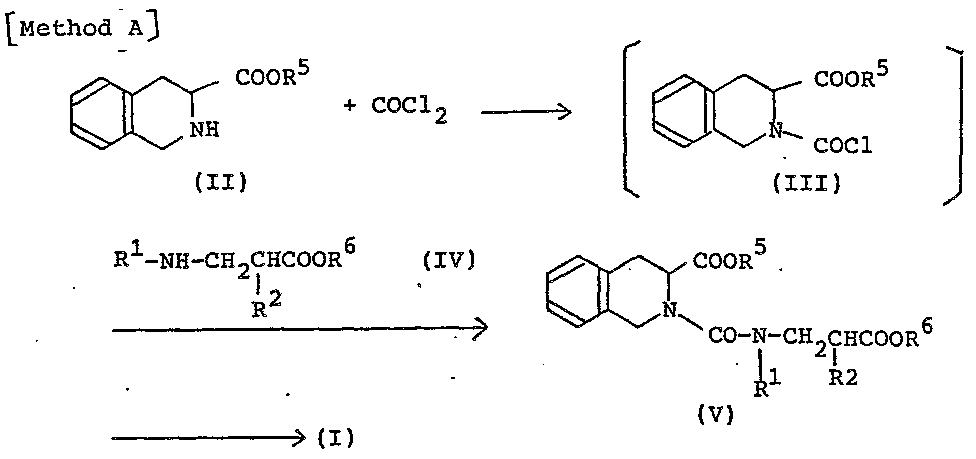 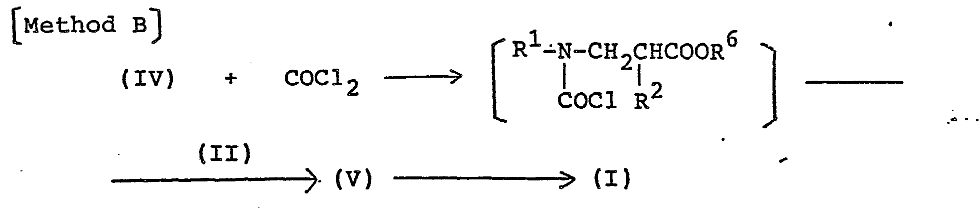 wherein R⁵ is lower alkyl or aralkyl, R⁶ is lower alkyl and R¹ and _{R}² are the same as defined above.

That is, according Method A, the compound (I) is prepared by the steps of i) reacting the 1,2,3,4-tetrahydroisoquinoline-3-carboxylic acid derivative (II) with phosgene to give the 2-chlorocarbonyl-1,2,3,4-tetrahydrosioquinoline-3-carboxylic acid derivative (III), (ii) condensing said compound (III) with the 3-aminopropionic acid derivative (IV) to give the 2-(N,N-disubstituted-carbamoyl)-1,2,3,4-tetrahydroisoquinoline-3-carboxylic acid derivative (V), and, if required, (iii) further subjecting the compound (V) to catalytic hydrogenation and/or hydrolysis.

On the other hand, according to Method B, the compound (I) is prepared by the steps of i) reacting the 3-aminopropionic acid derivative (IV) with phosgene to give the N-chlorocarbonyl-3-amino-propionic acid derivative, (ii) condensing said compound with the 1,2,3,4-tetrahydroisoquinoline-3-carboxylic acid derivative (II) to give the 2-(N,N-disubstituted- carbamoyl)-1,2,3,4-tetrahydrosioquinoline-3-carboxylic acid derivative (V), and, if required, (ii) further subjecting the compound (V) to catalytic hydrogenation and/or hydrolysis.

The first step in the above-mentioned Methods A or B, i.e., the reaction of the compound (II) or (IV) with phosgene, can be accomplished by treating said compound (II) or (IV) and phosgene in the presence or absence of an acid acceptor in a suitable solvent. Preferred examples of the group _{R}⁵ in the compound (II) include lower alkyl such as methyl, ethyl, propyl or butyl and aralkyl such as benzyl, phenethyl, p-methoxybenzyl, diphenylmethyl or triphenylmethyl; and preferred examples of the group R include lower alkyl such as methyl, ethyl, propyl or butyl. On. the other hand, preferred examples of the acid acceptor include organic tertiary amines such as triethylamine, tripropylamine, _{N}-methylmorpholine, pyridine and the like. Methylene chloride, chloroform, tetrahydrofuran and dioxane are suitable as the solvent. It is preferred to carry out the reaction at a temperature of 0° to -30°C, especially at a temperature below about -20°C. For example, the reaction is preferably carried out by dissolving phosgene in a solvent and then adding dropwise thereto at said temperature a solution containing the compound (II) or (IV) and the acid acceptor. After the reaction, the compound (I_{II}) or (VI) thus obtained can be used for the subsequent step without separating it from the reaction solution. If required, however, said compound (III) or (VI) may be isolated from said reaction solution by removing the excess of phosgene under reduced pressure.

The condensation reaction of the compounds (III) and (_{IV}) as well as the condensation reaction of the compounds (VI) and (II) can be conducted either in the presence or absence of an acid acceptor in a solvent. The acid acceptors and solvents exemplified in the first step as well as dimethylformamide are suitably used as said acid acceptor and solvent in this step. It is preferred to carry out the reaction at a temperature of 0° to 80°C, especially at 35° to 40°C.

The compound (I) in which both R³ and _{R}^{4.}are hydrogen atoms is prepared by further hydrolyzing the thus-obtained compound (V) to remove the groups R⁵ and R therefrom. Said hydrolysis is readily carried out by treating the compound (V) with an alkali agent in a solvent. Preferred examples of the alkali agent include alkali metal hydroxides such as sodium hydroxide or potassium hydroxide and alkali metal carbonates such as sodium carbonate or potassium carbonate. Water, lower alkanol (e.g., methanol, ethanol) or a mixture thereof are suitable as the solvent. The reaction proceeds smoothly at a temperature of about 0° to ₅0°C, especially at about 20" to 30°C.

On the other hand, when the group R⁵ is aralkyl, the compound (I) in which R⁴ is hydrogen atom is prepared by catalytic hydrogenation of the compound (V). This catalytic hydrogenation is carried out in the presence of a catalyst in a hydrogen gas atmosphere in a solvent. Preferred examples of the catalyst include palladium-black, palladium-carbon, platinum oxide and the like. Lower alkanols such as methanol, ethanol and propanol are suitable as the solvent. It is preferred to carry out the reacton at a temperature of about 20° to 40°C under one to 5 atmospheric pressures and under shaking. For example, said reaction proceeds smoothly at about 25°C under about 3 atmospheric pressure.

Further, when the group R⁵ is aralkyl, the compound (_{I}) in which both R³ and R⁴ are hydrogen atoms is prepared by further subjecting the compound (V).to catalytic hydrogenation and hydrolysis. These catalytic hydrogenation and hydrolysis can be carried out under the same conditions as described above.

When the compound (I) of the present invention has one or two carboxyl group(s) within its molecule, said compound may be readily converted to a pharmaceutically acceptable salt thereof in conventional manners, for example, by neutralization with an alkali agent or amine.

The starting compound (II) of the present invention involves two optical isomer, i.e., S- and R-isomers, due to the asymmetric carbon atom at the 3rd-position of isoquinoline skeleton. However, since all of the above-mentioned reactions of the invention can be carried out without racemization, the compound (I) of the present invention in an optically active form can be readily obtained by the use of the corresponding optically active isomer of the compound (II) as the starting material of the invention. If the compound (_{IV}) in the. form of a racemic mixture is used as the starting material in the above-mentioned reactions, the optically active compound (I) thus obtained is composed of the two stereoisomers which differ in the steric configuration of the carbon atom at the 2nd-position of 3-aminopropionyl moiety thereof, but these isomers may be readily separated into each components by silica gel chromatography using toluene-ethyl acetate as a solvent. Throughout the specification, the isomer which is eluted in early fractions of silica gel column chromatography is designated as "d-isomer" and the isomer which is eluted into late fractions thereof is designated as "B-isomer".

Moreover, if required, the starting compound (IV) of the invention in the form of an optically active isomer may be used in the above-mentioned reactions. Such optically active isomer of the compound (IV) may be obtained by optical resolution therof, i.e., by the steps of reacting the racemate of the compound (IV) with optically active N-benzyloxycarbonyl- phenylalanine in a solvent to form the diastereoisomeric salts thereof, and separating the diastereoisomeric salts into each components therof by selective crystallization. By said selective crystallization, less soluble enantiomer is recovered as crystals from the reaction mixture, and the more soluble one remains in the reaction mixture. If _{N}-benzyloxycarbonyl-D-phenylalanine is used as the resolving agent, the compound (IV) having R-configuration at the 2nd-position of the propionic moiety of the formula : -_{CH2}CH(_{R}²)COOR³ forms the less soluble diastereoisomeric salt. On the other hand, if N-benzyloxycarbonyl-L-phenylalanine is used as the resolving agent, the compound (IV) having S-configuration at the 2nd-position of said propionic moiety forms the less soluble diastereoisomeric salt. Ethyl acetate is suitable as the solvent, and the selective crystallization can be readily conducted by adding isopropylether to an ethyl acetate solution containing the diastereoisomeric salts of the compound (IV). It is preferred to use ethyl acetate in an amount of 3 to 5 ml per gram of the compound (IV). It is also preferred that isopropylether which is used to cause the selective crystallization of the diastereoisomeric salts is used in an amount of 0.5 to 2 ml per ml of ethyl acetate. After the optical resolution, the compound (IV) in free form can be recovered by treating the thus-obtained diastereoisomeric salt with an alkali agent (e.g., sodium or potassium carbonate, sodium bicarbonate, sodium or potassium hydroxide).

Alternatively, the optical resolution of the starting compound (IV) may be conducted by the use of an optically active phenylalanine amide. For example, the optically active enantiomer of the compound (IV) may be obtained by converting it into a compound of the formula: 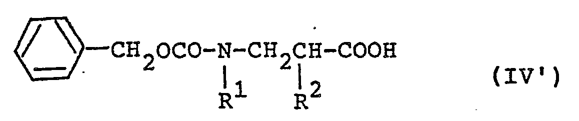 wherein _{R}¹ and R² are the same as defined above, reacting said compound (IV') with an optically active phenylalanine amide to form the diastereoisomeric salts thereof, separating the diastereoisomeric salts into each components by selective crystallization, esterifying the resultant optically active enantiomer with an alkanol of the formula: R⁶-OH (wherein _{R}⁶ is the same as defined above), and then removing benzyloxycarbonyl group therefrom. The selective crystallization of the diastereoisomeric salts is readily conducted by cooling an ether solution thereof or by adding n-hexane to an ethyl acetate solution thereof. On the other hand, the conversion of the compound (IV) into the compound (IV') can be carried out in conventional manners, for example, by reacting the former compound with benzyloxycarbonyl chloride and hydrolyzing it with an alkali agent such as sodium hydroxide. The esterification of an optically active enantiomer of the compound (IV') and the removal of benzyloxycarbonyl group therefrom can also be conducting in a manner known per se.

Concomitantly, among the starting compounds of the invention, the compound (II) is obtained by Pictet-Spengler's condensation reaction of phenylalanine and formaldehyde to give l,2,3,4-tetrahydroisoquinoline-3-carboxylic acid [Journal of The American Chemical Society Vol. 70, page 180 (1948)], followed by conventional esterification thereof. On the other hand, the compound (IV) is obtained by reacting an alkyl- or phenylalkyl-malonic acid monoethyl ester with formaldehyde and diethylamine [Journal of The Chemical Society of Japan Vol. 80, page 502.(1959)], and then reacting the resulting acrylic ester derivative with a primary amine [R.B.Wagner et al's Synthetic Organic Chemistry, page 673 (1961)].

### Example 1

A solution of 5.2 g of ethyl 2-n-octyl-3-ethylamino- propionate and 3.0 g of triethylamine in 50 ml of methylene chloride is added dropwise to 30 ml of a methylene chloride solution containing 2.6 g of phosgene. Said dropwise addition is carried out at about -30°C under stirring. The solution is stirred at the same temperature for 30 minutes and then condensed under reduced pressure. The residue obtained is dissolved in 20 ml of methylene chloride, and a solution of 5.4 g of benzyl (3S)-1,2,3,4-tetrahydrosioquinoline-3-carboxylate and 3.0 g of triethylamine in 50 ml of methylene chloride is added dropwise thereto at room temperature under stirring. The mixture is stirred at room temperature overnight. Then, the reaction mixture is washed with. dilute hydrochloric acid and an aqueous sodium bicarbonate solution, dried and condensed to remove solvent. 8.8 g of the oily residue thus obtained are purified by silica gel column chromatography (Solvent, toluene-ethyl acetate (20 : 1)), whereby benzyl (3S)-2-[N-((2R)-2-ethoxycarbonyl-n-decyl)-N-ethylcarbamoyl]-1,2,3,4-tetrahydroisoquinoline-3-carboxylate (α-isomer) is eluted into early fractions and benzyl (3S)-2-[N-((2S)-2-ethoxycarbonyl-n-decyl)-N-ethylcarbamoyl]-1,2,3,4-tetrahydroisoquinoline-3-carboxylate (B-isomer) into late fractions.

α-isomer Colorless surup Yield: 3.3 g (30.0 %) IR 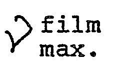 (cm⁻¹): 1730, ₁₆₅₀

B-isomer: Colorless surup Yield: 3.1 g (28.1 %) IR 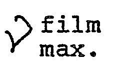 (cm⁻¹) ₁₇₃₀, ₁₆₅₀

### Example

(1) 2.2 g of benzyl (3S)-2-[N-((2R)-2-ethoxycarbonyl- n-decyl)-N-ethylcarbamoyl]-1,2,3,4-tetrahydroisoquinoline-3-carboxylate are dissolved in 50 ml of ethanol, and 50 mg of palladium-black are added thereto. The mixture is shaken at room temperature in hydrogen gas atmosphere for one hour under an atmospheric pressure. The catalyst is removed by filtration. 10 ml of water and 0.15 g of calcium hydroxide are added to the filtrate, and the aqueous mixture is stirred for one hour. Then, insoluble materials are removed by filtration, and the filtrate is condensed under reduced pressure. Crystalline precipitates are collected by filtration, washed with water and dried. 1.9 g of calcium (3S)-2-[N-((2R)-2-ethoxycarbonyl-n-decyl)-N-ethylcarbamoyl] - 1,2,3,4-tetrahydro-isoquinoline-3-carboxylate are thereby obtained as colorless powder. Yield: 99.2 %

M.p. 116 - 119°C 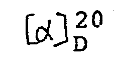 + 8.8°C(c=l, ethanol) IR 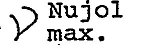 .(cm⁻¹): ₃₃₅0, 1730, 1640, 1605, 1565

Benzylamine salt: M.p. 87 - 92°C (recrystallized from isopropyl ether) 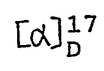 + 20.8° (c=l, ethanol)

(2). 2.94 g of benzyl (3S)-2-[N-((2S)-2-ethoxycarbonyl- n-decyl)-N-ethylcarbamoyl]-1,2,3,4-tetrahydroisoquinoline-3-carboxylate are treated in the same manner as described in paragraph (1). 2.4 g of calcium (3S)-2-[N-((2S-)-2-ethoxycarbonyl-n-decyl)-N-ethylcarbamoyl]-1,2,3,4-tetrahydroisoquinoline-3-carboxylate are thereby obtained as colorless powder. Yield: 93.8 %

M.p. 117 - 118°C 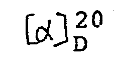 + 13.9° (c=l, ethanol) IR 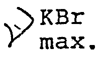 (cm⁻¹): 3400, 1730, 1640, 1610, 1560

Benzylamine salt:

M.p. 51 - 53°C (recrystallized from n-hexane) 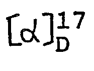 + 12.0° (c=l, ethanol)

### Example 3

(1) 1.0 g of benzyl (3S)-2-[N-((2R)-2-ethoxycarbonyl- n-decyl)-N-ethylcarbamoyl]-1,2,3,4-tetrahydroisoquinoline-3-carboxylate are dissolved in 30 ml of ethanol, and 30 mg of palladium-black are added thereto. The mixture is shaken at room temperature in hydrogen gas atmosphere for one hour under an atmospheric pressure. The catalyst is removed by filtration. A solution of 0.4 g of potassium hydroxide in 10 ml of water is added to the filtrate, and the aqeuous mixture is stirred at room temperature overnight. Then, the aqueous mixture is condensed under reduced pressure to remove ethanol, made acidic with hydrochloric acid and extracted with chloroform. The extract is dried and condensed to remove solvent. The residue thus obtained is then washed with a mixture of ether and n-hexane. 0.6 g of (3S)-2-[N-((2R)-2-carboxy-n-decyl)-Iv-ethylcarbamoyl]-1,2,3,4-tetrahydroisoquinoline-3-carboxylic acid is thereby obtained as colorless powder. Yield 76.4 %

M.p. 100 - 101°C (decomp.) IR 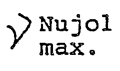 (cm⁻¹): 1730, 1600, 1585

(2) 1.2 g of benzyl (3S)-2-[N-((2S)-2-ethoxycarbonyl- n-decyl)-N-ethylcarbamoyl]-1,2,3,4-tetrahydroisoquinoline-3-carboxylate are dissolved in 30 ml of 90 % ethanol, and 0.6 g of potassium hydroxide is added thereto. The mixture is stirred at room temperature overnight. After the reaction, the mixture is condensed under reduced pressure to remove ethanol, made acidic with hydrochloric acid and extracted with chloroform. The extract is dried and condensed to remove solvent. Then, 30 ml of 90 % ethanol and 0.17 g of calcium hydroxide are added to the residue obtained, and the mixture is stirred for one hour. Insoluble materials are removed by filtration, and the filtrate is condensed under reduced pressure. Crystalline precipitates are collected by filtration, whereby 0.98 g of calcium (3S)-2-[N-((2S)-2-carboxy-n-decyl)-N-ethylcarbamoyl]-1,2,3,4-tetrahydroisoquinoline-3-carboxylate is obtained as colorless powder. Yield: 95.6 %

M.p. 208 - 211°C (decomp.) IR 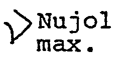 (cm⁻¹): 1580 (broad)

### Example 4

A solution of 10.1 g of ethyl 2-(2-phenylethyl)-3-ethylaminopropionate and 6.8 g of triethylamine in 30 ml of methylene chloride is added dropwise to 20 ml'of a methylene chloride solution containing 5.2 g of phosgene. Said dropwise addition is carried out at about -30°C under stirring. The solution is stirred at the-same temperature for 30 minutes and then condensed to dryness under reduced pressure. 30 ml of dimethylformamide are added to the residue obtained, and a solution of 10.7 g of benzyl (3S)-1,2,3,4-tetrahydroisoquinoline-3-carboxylate and 6.0 g of triethylamine in 30 ml of dimethylformamide is added dropwise thereto at room temperature under stirring. The mixture is stirred at room temperature overnight. Then, 100 ml of water are added to the reaction mixture, and said mixture is extracted with ether. The extract is washed with dilute hydrochloric acid and an aqueous sodium bicarbonate solution, dried and evaporated to remove solvent. 17.0 g of the oily residue thus obtained are purified by silica gel column chromatography (Solvent, toluene-ethyl acetate (20 : 1)), whereby benzyl (3S)-2-[N-((2R)-2-ethoxycarbonyl-4-phenyl-n-butyl)-N-ethylcarbamoyl]-1,2,3,4-tetrahydroisoquinoline-3-carboxylate (d-isomer) is eluted into early fractions and benzyl (3S)-2-[N-((2S)-2-ethoxycarbonyl-4-phenyl-n-butyl)-N-ethylcarbamoyl]-1,2,3,4-tetrahydroisoquinoline-3-carboxylate (B-isomer) into late fractions.

-isomer: Colorless syrup Yield: 6.3 g (29.0 %) IR 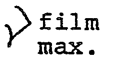 (cm⁻¹): 1730, 1650

B-isomers Colorless syrup Yield: 4.8 g (22,.1 %) IR 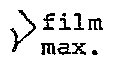 (cm⁻¹) : 1730, ₁₆₅₀

### Example 5

3.0 g of benzyl (3S)-2-[N-((2R)-2-ethoxycarbonyl-4-phenyl-n-butyl)-N-ethylcarbamoyl]-1,2,3,4-tetrahydroisoquinoline- . 3-carboxylate are dissolved in 50 ml of ethanol, and 0.1 g of 10 % palladium-carbon is added thereto. The mixture is shaken at room temperature in hydrogen gas atmosphere for ₃ hours under an atmospheric pressure. The catalyst is removed by filtration, and the filtrate is condensed to dryness. 2.4 g of (3S)-2-[N-((2R)-2-ethoxycarbonyl-4-phenyl-n-butyl)-N-ethylcarbamoyl]-1,2,3,4-tetrahydroisoquinoline-3-carboxylic acid are thereby obtained as colorless syrup. Yield: 96.0 % Benzylamine salt monohydrate: Colorless fine crystals (recrystallized from ethyl acetate) M.p. 107 - 111°C 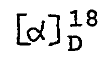 + 21.2° (c= 1, ethanol) IR 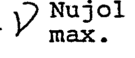 .(cm⁻¹) : 3550, 3450, 1730, 1650, 1615

Calcium salt monohydrate:

Colorless powder (recrystallized from ethanol-water) M.p. 147 - 148°C (decomp.) 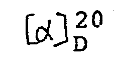 + 14.6° (c= 0.5, ethanol) IR 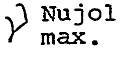 (cm⁻¹) : 1730, 1640, 1610

Cyclohexylamine salt: Colorless needles (recrystallized from acetone) M.p. 133 - 135°C 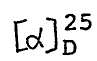 + 20.8°C (c= 1, ethanol) IR 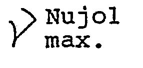 (cm⁻¹) : 1730, 1630, ₁₅₆0 max.

Lysine salt: Colorless powder (hygroscopic) M.p. ca. 63 - 65°C (decomp.) IR 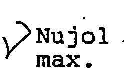 (cm⁻¹): 3450, 1730, 16₀0 (broad)

### Example 6

(₁) A mixture of 2.2 g of benzyl (3S)-2-[N-((2R)-2- ethoxycarbonyl-4-phenyl-n-butyl)-N-ethylcarbamoyl]-1,2,3,4-tetrahydroisoquinoline-3-carboxylate, 1.3 g of potassium hydroxide, 20 ml of ethanol and 10 ml of water is stirred at room temperature overnight. After the reaction, the mixture is evaporated under reduced pressure to remove ethanol. The residue is made acidic with hydrochloric acid and then extracted with ethyl acetate. The extract is washed with water, dried and condensed. The residue thus obtained is then crystallized with isopropyl ether. Crystalline precipitates are collected by filtration and washed with n-hexane. 0.92 g of (3S)-2-[N-((2R)-2-carboxy-4-phenyl-nlbutyl)-N-ethylcarbamoyl]-1,2,3,4-tetrahydroisoquinoline-3-carboxylic acid is thereby obtained as colorless powder. Yield: 53.5 % M.p. 114 - 115°C (decomp.) (recrystallized from ethyl acetate) 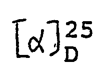 + 28.4° (c= 1, ethanol) IR 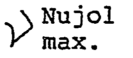 (cm⁻¹) : ₁₇₃₀, ₁₇₂₅, ₁590, 1580
(2) A mixture of 1.3 g of benzyl (3S)-2-[N-((2S)-2- ethoxycarbonyl-4-phenyl-n-butyl)-N-ethylcarbamoyl]-1,2,3,4-tetrahydroisoquinoline-3-carboxylate, 0.5 g of 10 % palladium-carbon and 50 ml of ethanol is shaken at room temperature in hydrogen gas atmosphere under an atmospheric pressure. After the reaction, the catalyst is removed by filtration, and the filtrate is condensed under reduced pressure. A solution of 0.3 g of potassium hydroxide in 10 ml of water is added to the residue, and the mixture is stirred at room temperature overnight. Then, the mixture is made acidic with hydrochloric acid and extracted with ethyl acetate. The extract is washed with water, dried and condensed to dryness. The residue obtained is dissolved in 50 ml of ethanol, 0.18 g of calcium hydroxide and 5 ml of water are added thereto, and said mixture is stirred for about 30 minutes. Then, insoluble materials are removed by filtration, and the filtrate is condensed under reduced pressure. The residue thus obtained is crystallized with 5 ml of water, whereby 0.75 g of calcium (3S)-2-[N-((2S)-2-carboxy-4-phenyl- n-butyl),-N-ethylcarbamoyl]-1,2,3,4-tetrahydroisoquinoline-3-carboxylate is obtained as colorless powder. Yield: 67.7 _{%} M.p. 235 - 239°C (decomp.) 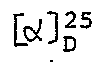 +30.0° (c= ₁, methanol)

### Example 7

4.1 g of ethyl 2-n-butyl-3-ethylaminoprbpionate, 5.4 g of benzyl (3S)-1,2,3,4-tetrahydroisoquinoline-3-carboxylate and 2.6 g of phosgene .are treated in the same manner as described in Example 1, Benzyl (3S)-2-N-((2R)-2-ethoxycarbonyl- n-hexyl)-N-ethylcarbamoyl]-1,2,3,4-tetrahydroisoquinoline-3-carboxylate (d-isomer) and benzyl (3S)-2-[N-((2S)-2-ethoxycarbonyl-n-hexyl)-N-ethylcarbamoyl]-1,2,3,4-tetrahydroisoquinoline-3-carboxylate (β-isomer) are obtained, respectively.

α-isomer: Colorless syrup Yiled: 2.5 g (25.0 %) IR 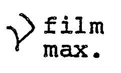 (cm⁻¹) : 1730, 1645 β-isomer: Colorless syrup Yield: 2.0 g (20.0 %) IR 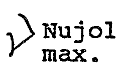 (cm⁻¹) : ₁₇₃₀, ₁₆₄₅

### Example 8

(1) 1.2 g of benzyl (3S)-2-[N-((2R)-2-ethoxycarbonyl- n-hexyl)-N-ethylcarbamoyl]-1,2,3,4-tetrahydroisoquinoline-3-carboxylate are treated in the same manner as described in Example 3-(1). 0.6 g of (3S)-2-[N-((2R)-2-carboxy-n-hexyl)-N-ethylcarbomoyl]-1,2,3,4-tetrahydroisoquinoline-3-carboxylic is obtained as colorless powder. Yield: 65.7 % M.p. 110 - 115°C (decomp.) IR 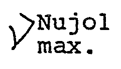 (cm⁻¹) : ₁₇₄₀, ₁₇₂₅, ₁₅₉₀, 1580
(2) 0.7 g of benzyl (3S)-2-[N-((2S)-2-ethoxycarbonyl- n-hexyl)-N-ethylcarbamoyl]-1,2,3,4-tetrahydroisoquinoline-3-carboxylate are treated in the same manner ap described in Example 3-(2). 0.3 g of calcium (3S)-2-[N-((2S)-2-carboxy-n-hexyl)-N-ethylcarbamoyl]-1,2,3,4-tetrahydroisoquinoline-3-carboxylate is obtained as colorless powder. Yield: 51.1 % M.p. 187 - 195°C (debomp.) IR 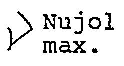 (cm⁻¹) : ₁₅₈₀ (broad)

### Example 9

₅.8 g of ethyl 2-n-decyl-3-ethylaminopropionate, 5.4 g of benzyl (3S)-1,2,3,4-tetrahydroisoguinoline-3-carboxylate and 2.6 g of phosgene are treated in the same manner as described in Example 1. Benzyl (3S)-2-[N-((2R)-2-ethoxycarbonyl-n-dodecyl)-N-ethylcarbamoyl]-1,2,3,4-tetrahydroisoquinoline-3-carboxylate (α-isomer) and benzyl (3_{S})-2-[N-((2S)-2-ethoxycarbonyl-n-dodecyl)-N-ethylcarbamoyl]-1,2,3,4-tetrahydroisoquinoline-3-carboxylate (β-isomer) are obtained, respectively.

α-isomer: Colorless syrup Yield: 4.8 g (41.0 %) IR 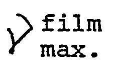 (cm⁻¹ ) : ₁₇₃₅, ₁₆₅₀ β-isomer: Colorless syrup Yield: 3.1 g (26.5 %) IR 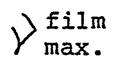 (cm⁻¹) : ₁₇₃₅, ₁₆₅₀

### Example 10

(1) 2.2 g of benzyl (3S)-2-[N-((2R)-2-ethoxycarbonyl- n-dodecyl)-N-ethylcarbamoyl]-1,2,3,4-tetrahydroisoquinoline-3-carboxylate are treated in the same manner as described in Example 6-(1). 1.1 g of (3S)-2-[N-((2R)-2-carboxy-n-dodecyl)-N-ethylcarbamoyl]-1,2,3,4-tetrahydroisoquinoline-3-carboxylic acid are obtained as colorless powder. Yield: 63.1 % M.p. 103 - 105°C (decomp.) 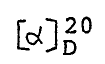 + 13.5° (c=l, methanol) IR 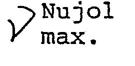 (cm⁻¹) : 1735, 1725, 1590, 1580
(₂) 2.0 g of benzyl (3S)-2-[N-((2S)-2-ethoxycarbonyl- n-dodecyl)-N-ethylcarbamoyl]-1,2,3,4-tetrahydroisoquinoline-3-carboxylate are treated in the same manner as described in Example 6-(1). 0.92 g of (3S)-2-[N-((2S)-2-carboxy-n-dodecyl)-N-ethylcarbamoyl]-1,2,3,4-tetrahydroisoquinoline- ₃-carboxylic acid is obtained as colorless powder. Yield: 53.6 % M.p. 157 - 162°C (decomp.) IR 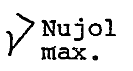 (cm⁻¹) : 1580 (broad)

### Example 11

6.4 g of ethyl 2-n-dodecyl-3-ethylaminopropionate, 5.4 g of benzyl (3S)-1,2,3,4-tetrahydroisoquinoline-3-carboxylate and 2.6 g of phosgene are treated in the same manner as described in Example 1. Benzyl (3S)-2-[N-((2R)-2-ethoxycarbonyl-n-tetradecyl)-N-ethylcarbamoyl]-1,2,3,4- tetrahydroisoquinoline-3-carboxylate (d-isomer) and benzyl (3S)-2-[N-((2S)-2-ethoxycarbenyl-n-tetradecyl)-N-ethylcarbamoyl]-1,2,3,4-tetrahydroisoquinoline-3-carboxylate (B-isomer) are obtained, respectively.

α-isomer: Colorless syrup Yield: 3.8 g (31.0 %) IR 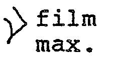 (cm⁻¹) : 1735, 1650

B-isomer: Colorless syrup Yield: 1.3 g (10.6 %) IR 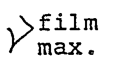 (cm⁻¹) : ₁₇₃₀, ₁₆₄₅

### Example 12

1.8 g of benzyl (3S)-2-[N-((2R)-2-ethoxycarbonyl-n-tetradecyl)-N-ethylcarbamoyl]-1,2,3,4-tetrahydroisoquinoline-3-carboxylate are treated in the same manner as described in Example 2-(1). 1.2 g of calcium (3S)-2-[N-((2R)-2-ethoxycarbonyl-n-tetradecyl)-N-ethylcarbamoyl]-1,2,3,4-tetrahydroisoquinoline-3-carboxylate are obtained as colorless powder. Yield: 75.5 %

M.p. 80 - 83°C IR 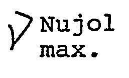 (cm⁻¹) : ₃₄₀₀, 1725, 1605, 1560 max.

### Example 13

(1) 1.6 g of benzyl (3S)-2-[N-((2R)-2-ethoxycarbonyl- n-tetradecyl)-N-ethylcarbamoyl]-1,2,3,4-tetrahydroisoquinoline-3-carboxylate are treated in the same manner as described in Example 6-(1). 0.98 g of (3S)-2-[N-((2R)-2-carb6xy-n-tetradegyl)-N-ethylcarbamoyl]-1,2,3,4-tetrahydroisoquinoline-3-carboxylic acid is obtained as colorless powder. Yield: 76.4 % M.p. 116 - 118°C (decomp.) IR 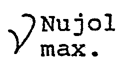 (cm⁻¹) : 1740, 1720, 1590, 1580 max.
(2) 1.0 g of benzyl (3S)-2-[N-((2S)-2-ethoxycarbonyl- n-tetradecyl)-N-ethylcarbamoyl]-1,2,3,4-tetrahydroisoquinoline-3-carboxylate is treated in the same manner as described in Example 6-(1), and the residue thus obtained is dissolved in 10 ml of ethanol. A solution of 0.5 g of L-lysine in one ml of water is added to the ethanol solution. The mixture is condensed under reduced pressure, and the residue is triturated with ether. 0.82 g of (3S)-2-[N-((2S)-2-carboxy- n-tetradecyl)-N-ethylcarbamoyl]-1,2,3,4-tetrahydroisoquinoline-3-carboxylic acid di-L-lysine salt is obtained as colorless powder. Yield: 63.9 % M.p. 187 - 190°C (decomp.) IR 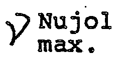 (cm⁻¹) : 1580 (broad)

### Example 14

3.8 g of ethyl 2-n-hexadecyl-3-ethylaminopropionate, 2.7 g of benzyl (3S)-1,2,3,4-tetrahydroisoquinoline-3-carboxylate and 1.3 g of phosgene are treated in the same manner as described in Example 1. Benzyl (3S)-2-[N-((2R)-2-ethoxycarbonyl-n-octadecyl)-N-ethylcarbamoyl]-1,2,3,4-tetrahydroisoquinoline-3-carboxylate (d-isomer) and benzyl (3S)-2-[N-((2S)-2-ethoxycarbonyl-n-octadecyl)-N-ethylcarbamoyl]-1,2,3,4-tetrahydroisoquinoline-3-carboxylate (B-isomer) are obtained, respectively.

d-isomer: Colorless syrup Yield: 1.2 g (17.9 %) IR 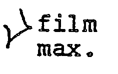 (cm⁻¹) : ₁₇₃₀, ₁₆₄₅ β-isomer: Colorless syrup Yield: 1.0 g (14.9 %) IR 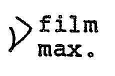 (cm⁻¹) : ₁₇₃₀, ₁₆₄₅

### Example 15

1.0 g of benzyl (3S)-2-[N-((2R)-2-ethoxycarbonyl- n-octadecyl)-N-ethylcarbamoyl]-1,2,3,4-tetrahydroisoquinoline-3-carboxylate is treated in the same manner as described in Example 6-(1). 0.36 g of (3S)-2-[N-((2R)-2-carboxy- n-octadecyl)-N-ethylcarbamoyl]-1,2,3,4-tetrahydroisoquinoline-3-carboxylic acid is obtained as colorless powder. Yield: 44.0 %

M.p. 92 - 94°C (decomp.) IR 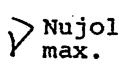 (cm⁻¹) : 1740, 1725, ₁₅₉₅, ₁₅₈₀

### Example 16

4.5 g of ethyl 2-benzyl-3-methylaminopropionate, 5.4 g of benzyl (3S)-1,2,3,4-tetrahydroiosquinoline-3-carboxylate and 2.6 g of phosgene are treated in the same manner as described in Example 1. 9.8 g of benzyl (3S)-2-[N-(2- ethoxycarbonyl-3-phenylpropyl)-N-methylcarbamoyl]-l,2,3,4-tetrahydrosioquinoline-3-carboxylate (a mixture of α- and B-isomers)are obtained as colorless syrup. Yield: 94.3 %

IR 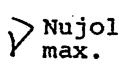 (cm⁻¹) : ₁₇₃0, 1645

### Example 17

1.5 g of benzyl (3S)-2-[N-(2-ethoxycarbonyl-3-phenylpropyl)-N-methylcarbamoyl]-1,2,3,4-tetrahydroisoquinoline-3-carboxylate obtained in Example 16 are treated in the same manner as described in Example 5, and the residue is dissolved in 10 ml of ethanol. A solution of 0.43 g of L-lysine in one ml of water is added to the ethanol solution. The mixture is condensed under reduced pressure, and the residue is triturated with ether. 1.4 g of (3S)-2-[N-(2-ethoxycarbonyl-3-phenylpropyl)-N-methylcarbamoyl] -1,2,3,4-tetrahydroisoquinoline-3-carboxylic acid L-lysine salt are obtained as colorless powder. Yield: 84.2 %

M.p. 66 - 70°C (decomp.)

Cyclohexylamine salt: Colorless needles (recrystallized from acetone) M.p. 133 - 136°C

### Example 18

1.5 g of benzyl (3S)-2-[N-(2-ethoxycarbonyl-3-phenylpropyl)-N-methylcarbamoyl]-1,2,3,4-tetrahydroisoquinoline-3-carboxylate obtained in Example 16 are treated in the same manner as described in Example 3-(2). 0.85 g of calcium (3S)-2-[N-(2-carboxy-3-phenylpropyl)-N-methylcarbamoyl]-1,2,3,4-tetrahydroisoquinoline-3-carboxylate is obtained as colorless powder. Yield: 67.1 %

M.p. 242 - 246°C (decomp.)

### Example 19

4.8 g of ethyl 2-benzyl-3-ethylaminopropionate, 5.4 g of benzyl (3S)-1,2,3,4-tetrahydroisoquinoline-3-carboxylate-and 2.6 g of phosgene are treated in the same manner as described in Example 1. Benzyl (3S)-2-[N-((2R)-2-ethoxycarbonyl-3-phenylpropyl]-N-ethylcarbamoyl]-1,2,3,4-tetrahydroisoquinoline-3-carboxylate (d-isomer)and benzyl (3S)-2-[N-((2S)-2-ethoxycarbonyl-3-phenylpropyl)-N-ethylcarbamoyl]-1,2,3,4-tetrahydroisoquinoline-3-carboxylate (8-isomer) are obtained, respectively.

α-isomer: Colorless syrup Yield: 2.1 g (19.7 %) IR 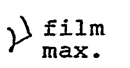 (cm⁻¹) : ₁₇₃0, 1645

B-isomer: Colorless syrup Yield: 1.6 g (15.0 %)

### Example 20

(1) 0.8 g of benzyl (3S)-2-[N-((2R)-2-ethoxycarbonyl-3-phenylpropyl)-N-ethylcarbamoyl]-1,2,3,4-tetrahydroisoquinoline-3-carboxylate is treated in the same manner as described in Example 3-(1). 0.36 g of (3S)-2-[N-((2R)-2-carboxy-3-phenylpropyl)-N-ethylcarbamoyl]-1,2,3,4-tetrahydroisoquinoline-3-carboxylic acid is obtained as colorless powder. Yield: 58.0 _{%}

M.p. 112 - 114°C IR 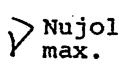 (cm⁻¹) : 1740, 1730, ₁6₀₅, ₁₅₉₀, ₁₅₇₀

(2) 1.2 g of benzyl (3S)-2-[N-((2S)-2-ethoxycarbonyl-3-phenylpropyl)-N-ethylcarbamoyl]-1,2,3,4-tetrahydroisoquinoline-3-carboxylate are treated in the same manner as described in Example 3-(2). 0.45 g of calcium (3S)-2-[N-((2S)-2-carboxy-3-phenylpropyl)-N-ethylcarbamoyl]-1,2,3,4-tetrahydroisoquinoline-3-carboxylate is obtained as colrless powder. Yield: 44.2 %

M.p. 215 - 219°C (decomp.) IR 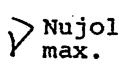 (cm⁻¹) : ₁₅₈0 (broad)

### Example 21

3.0 g of ethyl 2-benzyl-3-isopropylaminopropionate, 3.2 g of benzyl (3S)-1,2,3,4-tetrahydroisoquinoline-3-carboxylate and 1.5 g of phosgene are treated in the same manner as described in Example 1. Benzyl (3S)-2-[N-((2R)-2-ethoxycarbonyl-3-phenylpropyl)-N-isopropylcarbamoyl]-1,2,3,4-tetrahydroisoquinoline-3-carboxylate (d-isomer) and beznyl (3S)-2-fN-((2S)-2-ethoxycarbonyl-3-phenylpropyl)-N-isopropylcarbamoyl]-1,2,3,4-tetrahydroisoquinoline-3-carboxylate (B-isomer) are obtained, respectively.

α-isomer : Colorless syrup Yield: 2.1 g (32.3 %) IR 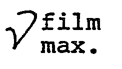 (cm⁻¹) : 1730, 1645

β-isomer : Colorless syrup Yield: 1.7 g (26.2 %) IR 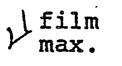 (cm⁻¹) : 1730, 1645

### Example 22

(1) 1.3 g of benzyl (3S)-2-[N-((2R)-2-ethoxycarbonyl-3-phenylpropyl)-N-isopropylcarbamoyl]-1,2,3,4-tetrahydroisoquinoline-3-carboxylate are treated in the same manner as described in Example 3-(2). 0.45'g of calcium (3S)-2-[N-((2R)-2-carboxy-3-phenylpropyl)-N-isopropylcarbamoyl]-1,2,3,4-tetrahydroisoquinoline-3-carboxylate is obtained as colorless powder. Yield: 40.6 %

M.p. 220 - 226°C (decomp.) IR 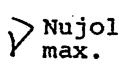 (cm⁻¹) : 1580 (broad)

(2) 1.0 g of benzyl (3S)-2-f_{N}-((2S)-2-ethoxycarbonyl-3-phenylpropyl)-N-isopropylcarbamoyl]-1,2,3,4-tetrahydroisoquinoline-3-carboxylate is treated in the same manner as described in Example 3-(2). 0.41 g of calcium (3S)-2-[N-((2S)-2-carboxy-3-phenylpropyl)-N-isopropylcarbamoyl]-1,2,3,4-tetrahydroisoquinoline-3-carboxylate is obtained as colorless powder. Yield: 48.1 %

M.p. 217 - 225°C (decomp). IR 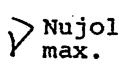 (cm⁻¹) : 1580 (broad)

### Example 23

2.7 g of ethyl 2-(2-phenylethyl)-3-isopropylamino- propionate, 2.7 g of benzyl (3S)-1,2,3,4-tetrahydroisoquinoline-3-carboxylate and 1.3 g of phosgene are treated in the same manner as described in Example 1. Benzyl (3S)-2-[N-((2R)-2-ethoxycarbonyl-4-phenyl-n-butyl)-N-isopropylcarbamoyl]-1,2,3,4-tetrahydroisoquinoline-3-carboxylate (α-isomer) and benzyl (3S)-2-N-((2S)-2-ethoxycarbonyl-4-phenyl-n-butyl)-N-isopropylcarbamoyl-1,2,3,4-tetrahydroisoquinoline-3-carboxylate (B-isomer) are obtained, respectively.

α-isomer: Colorless syrup Yield: 2.2 g (39.1 %) IR 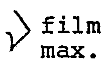 (cm⁻¹) : ₁₇₃₀, 1640 B-isomer

Colorless syrup Yield: 1.8 g (32.0 %) IR 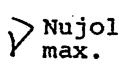 (cm⁻¹) : ₁₇₃₀, ₁₆₄₅

### Example 24

(1) 1.0 g of benzyl (3S)-2-[N-((2R)-2-ethoxycarbonyl-4-phenyl-n-butyl)-N-isopropylcarbamoyl]-1,2,3,4-tetrahydroisoquinoline-3-carboxylate is treated in the same manner as described in Example 2-(1). 0.5 g of calcium (3S)-2-[N-((2R)-2-ethoxycarbonyl-4-phenyl-n-butyl)-N-isopropylcarbamoyl]-1,2,3,4-tetrahydroisoquinoline-3-carboxylate is obtained as colorless powder. Yield: 57.3 _{%}

M.p. 92 - 95°C (decomp.) IR 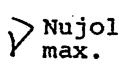 (cm⁻¹) ; ₃₄₀0 (broad), 1730, 1580 (broad)

(2) 0.75 g of benzyl (3S)-2-[N-((2S)-2-ethoxycarbonyl-4-phenyl-n-butyl)-N-isopropylcarbamoyl]-1,2,3,4-tetrahydroisoquinoline-3-carboxylate is treated in the same manner as described in Example 17. 0.65 g of (3S)-2-[N-((2S)-2- ethoxycarbonyl-4-phenyl-n-butyl)-N-isopropylcarbamoyl]-l,2,3,4-tetrahydroisoquinoline-3-carboxylic acid L-lysine salt is obtained as colorless powder. Yield: 78.7 _{%}

M.p. 76 - 79°C (decomp.) IR 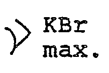 (cm⁻¹) : 3400 (broad), 1730, 1590 (broad)

### Example 25

(1') 1.0 g of benzyl (3S)-2-[N-((2R)-2-ethoxycarbonyl-4-phenyl-n-butyl)-N-isopropylcarbamoyl]-1,2,3,4-tetrahydroisoquinoline-3-carboxylate is treated in the same manner as described in Example 3-(1). 0.46 g of (3S)-2-[N-((2R)-2-carboxy-4-phenyl-n-butyl)-N-isopropylcarbamoyl]-1,2,3,4-tetrahydroisoquinoline-3-carboxylic acid is obtained as colorless powder. Yield: 58.4 % M.p. 120 - 122°C (decomp.) IR 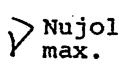 (cm⁻¹) : 1735, 1590, 1575

(2) 0.75 g of benzyl (3S)-2-[N-((2S)-2-ethoxycarbonyl-4-phenyl-n-butyl)-N-isopropylcarbamoyl]-1,2,3,4-tetrahydroisoquinoline-3-carboxylate is treated in the same manner as described in Example 3-(2). 0.4 g of calcium (3S)-2-[N-((2S)-2-carboxy-4-phenyl-n-butyl)-N-isopropylcarbamoyll-1,2,3,4-tetrahydroisoquinoline-3-carboxylate is obtained as colorless powder. Yield: 62.3 %

M.p. 238 - 240°C (decomp.) IR 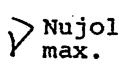 (cm⁻¹) : ₃₃₅₀ (broad), 1590 (broad)

### Example 26

2.7 g of ethyl 2-(3-phenylpropyl)-3-ethylaminopropionate, 2.7 g of benzyl (3S)-1,2,3,4-tetrahydroisoquinoline-3-carboxylate and 1.3 g of phosgene are treated in the same manner as described in Example 1. Benzyl (3S)-2-[N-((2R)-2-ethoxycarbonyl-5-phenyl-n-pentyl)-N-ethylcarbamoyl]-1,2,3,4-tetrahydroisoquinoline-3-carboxylate (d-isomer) and benzyl (3S)-2-[N-((2S)-2-ethoxycarbonyl-5-phenyl-n-pentyl)-N-ethylcarhumoyl]-1,2,3,4-tetrahydroisoquinoline-3-carboxylate (B-isomer) are obtained, respectively.

α-isomer: Colorless syrup Yield: 1.7 g (30.2 %) IR 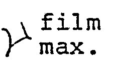 (cm⁻¹) : 1₇3₅, ₁₆₅₀

β-isomer : Colorless syrup Yield: 1.1 g (19.G %) IR 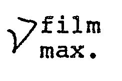 (cm⁻¹) : ₁₇₃₀, ₁₆₄₀

### Example 27

(1) 1.5 g of benzyl(3S)-2-[N-((2R)-2-ethoxycarbonyl-5-phenyl-n-pentyl)-N-ethylcarbamoyll-l,2,3,4-tetrahydroisoquinoline-3-carboxylate are treated in the same manner as described in Example 3-(2). 0.91 g of calcium (3S)-2-[N-((2R)-2-carboxy-5-phenyl-n-pentyl)-N-ethylcarbamoyl]-1,2,3,4-tetrahydroisoquinoline-3-carboxylate is obtained as colorless powder. Yield: 70.9 %

M.p. 232°C (decomp.) IR 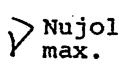 (cm⁻¹) : 1575 (broad)

(2) 0.8 g of benzyl (3S)-2-[N-((2S)-2-ethoxycarbonyl-5-pheneyl-n-pentyl)-N-ethylcarbamoyl]-1,2,3,4-tetrahydroisoquinoline-3-carboxylate is treated in the same manner as described in Example 3-(2). 0.83 g of (3S)-2-[N-((2S)-2-carboxy-5-phenyl-n-pentyl)-N-ethylcarbamoyl]-1,2,3,4-tetrahydroisoquinoline-3-carboxylic acid di-L-lysine salt is obtained as colorless powder. Yield: 79.2 %

M.p. 180 - 184°C (decomp.) IR 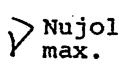 (cm⁻¹) : 1600 (broad)

### Example 28

2.3 g of ethyl 2-methyl-3-phenethylaminopropionate, 2.7 g of benzyl (3S)-1,2,3,4-tetrahydroisoquinoline-3-carboxylate, 1.3 g of phosgene, 3.0 g of triethylamine, 30 ml of methylene chloride and 30 ml of dimethylformamide are treated in the same manner as described in Example 4. 2.9 g of benzyl (3S)-2-[N-(2-ethoxycarbonylpropyl)-N-phenethyl- carbamoyl]-1,2,3,4-tetrahydroisoquinoline-3-carboxylate (a mixture of α- and B-isomers) are obtained as colorless syrup. Yield: 55.8 % IR 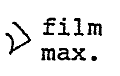 (cm⁻¹) : 1735, 1645

### Example 29

1.6 g of benzyl (3S)-2-[N-(2-ethoxycarbonylpropyl)-N-phenethylcarbamoy]-1,2,3,4-tetrahydroisoquinoline-3-carboxylate obtained in Example 28 are treated in the same manner as described in Example 5, and the product is then treated with 0.31 g of cyclohexylamine. 1.1 g of (3S)-2-[N-(2-ethoxycarbonylpropyl)-N-phenethylcarbamoyl]-1,2,3,4-tetrahydroisoquinoline-3-carboxylic acid cyclohexylamine salt are obtained as colorless powder. Yield: 67.6 % M.p. 149 - 151°C
IR 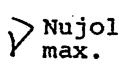 (cm⁻¹) : 1730, ₁₆₄₀, 1₅₆₀

### Example 30

1.0 g of benzyl (3S)-2-[N-(2-ethoxycarbonylpropyl)-N-phenethylcarbamoyl]-1,2,3,4-tetrahydroisoquinoline-3-carboxylate obtained in Example 28 is treated in the same'manner as described in Example 13-(2). 1.2 g of (3S)-2-[N-(2-carboxypropyl)-N-phenethylcarbamoyl]-1,2,3,4-tetrahydroisoquinoline-3-carboxylic acid di-L-lysine salt.are obtained as colorless powder. Yield: 87.9 % M.p. 85°C (decomp.) IR 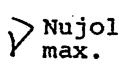 (cm⁻¹) : ₁₆₀₀ (broad)

### Example 31

4.9 g of ethyl 2-(2-phenethyl)-3-phenethylamino- propionate, 4.0 g of benzyl (3S)-1,2,3,4-tetrahydroisoquinoline-3-carboxylate, 1.9 g of phosgene, 4.6 g of triethylamine, 80 ml of methylene chloride and 30 ml of dimethylformamide are treated in the same manner as described in Example 4. Benzyl (3S)-2-[N-((2R)-2-ethoxycarbonyl-4-phenyl-n-butyl))-N-phenethylcarbamoyl]-1,2,3,4-tetrahydroisoquinoline-3-carboxylate (d-isomer) and benzyl (3S)-2-[N-((2S)-2-ethoxycarbonyl-4-phenyl-n-butyl)-N-phenethylcarbamoyl]-1,2,3,4-tetrahydroisoquinoline-3-carboxylate (B-isomer) are obtained, respectively.

α-isomer: Colorless syrup Yield: 3.8 g (41.0 %) IR 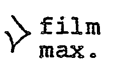 (cm⁻¹) : ₁₇₃₀, 1640

β-isomer Colorless syrup Yield: 2.4 g (25.9 %)

### Example 32

(1) 2.0 g of benzyl (3S)-2-[N-((2R)-2-ethoxycarbonyl-4-phenyl-n-butyl)-N-phenethylcarbamoyl]-1,2,3,4-tetrahydroisoquinoline-3-carboxylate are treated in the same manner as described in Example 13-(2). 1.9 g of (3S)-2-[N-((2R)-2-carboxy-4-phenyl-n-butyl)-N-phenethylcarbamoyl]-1,2,3,4-tetrahydroisoquinoline-3-carboxylic acid di-L-lysine salt are obtained as colorless powder. Yield: 74.3 %

M.p. 77°C (decomp.) IR 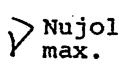 (cm⁻¹) : ₁₆₀0 (broad)

(2) 1.4 g of benzyl (3S)-2-[N-((2S)-2-ethoxycarbonyl-4-phenyl-n-butyl)-N-phenethylcarbamoylJ-1,2,3,4-tetrahydroisoquinoline-3-carboxylate are treated in the same manner as described in Example 3-(2). 0.84 g of calcium (3S)-2-[N-((2S)-2-carboxy-4-phenyl-n-butyl)-N-phenethylcarbamoyl]-1,2,3,4-tetrahydroisoquinoline-3-carboxylate is obtained as colorless powder. Yield: 69.2 %

M.p. 212 - 214°C (decomp.) IR 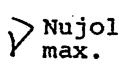 (cm⁻¹) : 1580 (broad)

### Example 33

A solution of 5.4 g of benzyl (3S)-1,2,3,4-tetrahydroisoquinoline-3-carboxylate and 3.0 g of triethylamine in 30 ml of methylene chloride is added dropwise to 30 ml of a methylene chloride solution containing 2.6 g of phosgene. Said dropwise addition is carried out at about -30°C under stirring. The solution is stirred at the same temperature for 30 minutes and then condensed under reduced pressure. The residue obtained is dissolved in 30 ml of methylene chloride, and a solution of 3.8 g of ethyl 2-isopropyl-3-ethylaminopropionate and 3.0 g of triethylamine in 30 ml of methylene chloride is added dropwise thereto at room temperature under stirring. The mixture is stirred at room temperature overnight. Then, the reaction mixture is treated in the same manner as described in Example 1. Benzyl (3S)-2-EN-((2R)-2-ethoxycarbonyl-3-methyl-n-butyl)-N-ethylcarbamoyl]-1,2,3,4-tetrahydroisoquinoline-3-carboxylate (α-isomer) and benzyl (3S)-2-N-((2S)-2-ethoxycarbonyl-3-methyl- n-butyl)-N-ethylcarbamoyl]-1,2,3,4-tetrahydroisoquinoline-3-carboxylate (β-isomer) are obtained, respectively.

d-isomer: Colorless syrup Yield: 2.1 g (21.6 _{%}) IR 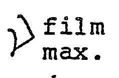 . (cm⁻¹) : ₁₇₃₀, 1645

β-isomer: Colorless syrup Yield: 1.6 g (16.5 %) IR 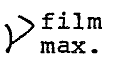 (cm⁻¹) : ₁₇₃₀, ₁₆₄₅

### Example 34

(1) 1.0 g of benzyl (3S)-2-[N-((2R)-2-ethoxycarbonyl-3-methyl-n-butyl)-N-ethylcarbamoyl]-1,2,3,4-tetrahydroisoquinoline-3-carboxylate is treated in the same manner as described in Example 2-(1), and the product is then treated with 0.3 g of L-lysine. 0.5 g of (3S)-2-[N-((2R)-2-ethoxycarbonyl-3-methyl-n-butyl)-N-ethylcarbamoyl]-1,2,3,4-tetrahydroisoquinoline-3-carboxylic acid L-lysine salt is obtained as colorless powder. Yield: 44.8 %

M.p. 116 - 117°C (decomp.) IR 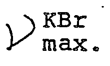 (cm⁻¹) : 3280, 1730, 1630, 1550

(2) 1.3 g of benzyl (3S)-2-[N-((2S)-2-ethoxycarbonyl-3-methyl-n-butyl)-N-ethylcarbamoyl]-1,2,3,4-tetrahydroisoquinoline-3-carboxylate are treated in the same manner as described in Example 2-(1). 0.6 g of calcium (3S)-2-[N-((2S)-2-ethoxycarbonyl-3-methyl-n-butyl)-N-ethylcarbamoyl]-1,2,3,4-tetrahydroisoquinoline-3-carboxylate is obtained as colorless powder. Yield: 54.2 %

M.p. 141 - 144°C (decomp.) IR 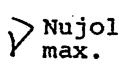 (cm⁻¹): ₃₃₈₀, ₁₇₃₀, ₁₆₅₅, ₁₆₀₅, ₁₅6₀ (broad)

### Example 35

(1) 1.1 g of benzyl (3S)-2-[N-((2R)-2-ethoxycarbonyl-3-methyl-n-butyl)-N-ethylcarbamoyl]-1,2,3,4-tetrahydroisoquinoline-3-carboxylate are treated in the same manner as described in Example 13-(2). 0.65 g of (3S)-2-[N-((2R)-2-carboxy-3-methyl-n-butyl)-N-ethylcarbamoyl]-1,2,3,4-tetrahydroisoquinoline-3-carboxylic acid di-L-lysine salt is obtained as colorless powder. Yield: 43.3%

M.p. 208 - 212°C (decomp.) IR 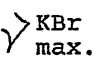 (cm⁻¹) : 1580 (broad)

(2) 0.6 g of benzyl (3S)-2-[N-((2S)-2-ethoxycarbonyl-3-methyl-n-butyl)-N-ethylcarbamoyl]-1,2,3,4-tetrahydroisoquinoline-3-carboxylate is treated in the same manner as described in paragraph (1). 0.51 g of (3S)-2-[N-((2S)-2-carboxy-3-methyl-n-butyl)-N-ethylcarbamoyl]-1,2,3,4-tetrahydroisoquinoline-3-carboxylic acid di-L-lysine salt is obtained as colorless powder. Yield: 62.4 % M.p. 172°C (decomp.)

### Example 36

5.4 g of benzyl (3S)-1,2,3,4-tetrahydroisoquinoline₋ 3-carboxylate, 3.0 g of methyl 2-methyl-3-ethylaminopropionate and ₂.6 g of phosgene are treated in the same manner as described in Example 33. Then, the reaction mixture is washed with diluted hydrochloric acid and aqueous sodium bicarbonate, dried and condensed to remove solvent. 7.7 g of benzyl (3S)-2-[N-(2-methoxycarbonylpropyl)-N-ethylcarbamoyl] -1,2,3,4-tetrahydroisoquinoline-3-carboxylate (a mixture of α- and 8- isomers) are obtained as colorless syrup. Yield: 86.9 % IR 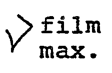 (cm⁻¹) : ₁₇₄₀, ₁₆₄₅

### Example 37

1.7 g of benzyl (3S)-2-[N-(2-methoxycarbonylpropyl)-N-ethylcarbamoyl]-1,2,3,4-tetrahydroisoquinoline-3-carboxylate obtained in Example 36 is treated in the same manner as described in Example 5, and the product is then treated with 0.4 g ofcyclohexylamine. 0.9 g of (3S)-2-[N-(2-methoxy- carbonylprcpyl)-N-ethylcarbamoyl]-1,2,3,4-tetrahydroisoquinoline-3-carboxylic acid cyclohexylamine salt is obtained as colorless crystals. Yield: 51.9 % M.p. 142 - 145°C IR 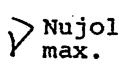 (cm⁻¹) : 1740, 1635, 1555

### Example 38

1.5 g of benzyl (3S)-2-[N-(2-methoxycarbonylpropyl)-N-ethylcarbamoyl]-1,2,3,4-tetrahydroisoquinoline-3-carboxylate obtained in Example 36 are treated in the same manner as described in Example 3-(2). 0.99 g of calcium (3S)-2-[N-(2-carhoxylpropyl)-N-ethylcarbamoyl]-1,2,3,4-tetrahydroisoquinoline-3-carboxylate is obtained as colorless powder. Yield: 78.1 %

M.p. > 260°C IR 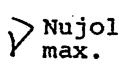 (cm⁻¹) : 3380, 16₀0, ₁₅₇₀, ₁₅₄₀

### Example 39

5.4 g of benzyl (3S)-1,2,3,4-tetrahydroisoquinoline-3-carboxylate, 4.8 g of ethyl 2-(2-phenylethyl)-3-methyl- aminopropionate and 2.6 g of phosgene are treated in the same manner as described in Example 33. 9.1 g of benzyl (3S)-2-[N-(2-ethoxycarbonyl-4-phenyl-n-butyl)-N-methylcarbamoyl]-1,2,3,4-tetrahydroisoquinoline-3-carboxylate (a mixture of d- and β-isomers) are obtained as colorless syrup. Yield: 85.2 % IR 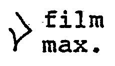 (cm⁻¹) : 1730, 1645

### Example 40

3.1 g of benzyl (3S)-2-[N-(2-ethoxycarbonyl-4-phenyl-. n-butyl)-N-methylcarbamoyl]-1,2,3,4-tetrahydroisoquinoline-3-carboxylate obtained in Example 39 are treated in the same manner as described in Example 2-(1). 2.4 g of calcium (3S)-2-[N-(2-ethoxycarbonyl-4-phenyl-n-butyl)-N-methylcarbamoyl]-1,2,3,4-tetrahydroisoquinoline-3-carboxylate are obtained as colorless powder. Yield: 89.5 %

M.p. 129 - 131°C (decomp.) IR 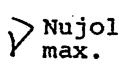 (cm⁻¹) : 3300, 1730, 1645, 1610, 1570

Cyclohexylamine salt: Colorless needles (recrystallized from isopropanol) - M.p. 147 - 148°C (decomp.) 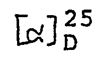 + 18.8° (c= 1, ethanol) IR 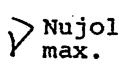 (cm⁻¹) : 1730, 164₀, ₁₆₁₀, ₁₅₆₅

### Example 41

1.9 g of benzyl (3S)-2-[N-(2-ethoxycarbonyl-4-phenyl- n-butyl)-N-methylcarbamoyl]-1,2,3,4-tetrahydroisoquinoline-3-carboxylate obtained in Example 39 are treated in the same manner as described in Example 3-(2). 1.38 g of calcium (3S)-2-[N-(2-carboxy-4-phenyl-n-butyl)-N-methylcarbamoyl]-1,2,3,4-tetrahydroisoquinoline-3-carboxylate are obtained as colorless powder. Yield: 85.6 % M.p. 254 - 258°C (decomp.) IR 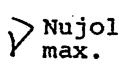 (cm⁻¹) : 3400, 1590 (broad)

### Example 42

2.7 g of ethyl 2-n-octyl-3-ethylaminopropionate, 2.1 g of ethyl (3S)-1,2,3,4-tetrahydroisoquinoline-3-carboxylate and 1.3 g of phosgene are treated in the same manner as described in Example 1. Ethyl (3S)-2-[N-((2R)-2-ethoxycarbonyl- n-decyl)-N-ethylcarbamoyl]-1,2,3,4-tetrahydroisoquinoline-3-carboxylate ((α-isomer) and ethyl (3S)-2-[N-((2S)-2-ethoxycarbonyl-n-decyl)-N-ethylcarbamoyl]-1,2,3,4-tetrahydroisoquinoline-3-carboxylate (B-isomer) are obtained, respectively.

d-isomer: Colorless syrup Yiled: 1.6 g (32.0 %) IR 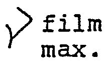 (cm⁻¹) : 1730, 1645

B-isomer: Colorless syrup Yield: 1.4 g (28.0 %) IR 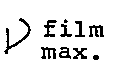 (cm⁻¹) : 1730, 1645

### Example 43

4.7 g of ethyl 2-n-hexyl-3-ethylaminopropionate, 5.4 g of benzyl (3S)-1,2,3,4-tetrahydroisoquinoline-3-carboxylate, 2.6 g of phosgene, 6.0 g of triethylamine, 30 ml of methylene chloride and 20 ml of dimethylformamide are treated in the same manner as described in Example 4. Benzyl (3S)-2-[N-((2R)-2-ethoxycarbonyl-n-octyl)-N-ethylcarbamoyl]-1,2,3,4-tetrahydroisoquinoline-3-carboxylate (d-isomer) and benzyl (3S)-2-[N-((2S)-2-ethoxycarbonyl-n-octyl)-N-ethylcarbamoyl]-1,2,3,4-tetrahydroisoquinoline-3-carboxylate are obtained, respecitvely.

α-isomer: Colorless syrup Yield: 3.0 g (28.4 %) IR 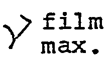 (cm⁻¹) : 1730, 1650

β-isomer: Colorless syrup Yield: 2.1 g (19.9 %) ^{I}R ν^{film}ₘₐₓ. (cm⁻¹) : 1730, 1650

### Example 44

1.7 g of benzyl (3S)-2-[N-((2R)-2-ethoxycarbonyl-n-octyl)-N-ethylcarbamoyl]-1,2,3,4-tetrahydroisoquinoline-3-carboxylate, 50 ml of ethanol, 0.5 g of 10 % palladium-carbon and 0.12 g of calcium hydroxide are treated in the same manner as described in Example 2-(1). 1.0 g of calcium (3S)-2-[N-((2R)-2-ethoxycarbonyl-n-octyl)-N-ethylcarbamoyl]-1,2,3,4-tetrahydroisoquinoline-3-carboxylate is obtained as colorless powder. Yield: 68.1 %

M.p. 102°C (decomp.) IR 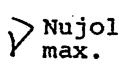 (cm⁻¹) : ₁₇₃₅, ₁₆₄₀, ₁₆₁₀, ₁₅₇₀

### Example 45

1.2 g of benzyl (3S)-2-[N-((2R)-2-ethoxycarbonyl-n-octyl)-N-ethylcarbamoyl]-1,2,3,4-tetrahydroisoquinoline-3-carboxylate are treated in the same manner as described in Example 6-(1). 0.62 g of (3S)-2-[N-((2R)-2-carboxy-n-octyl)-N-ethylcarbamoyl]-1,2,3,4-tetrahydroisoquinoline-3-carboxylic acid is obtained as colorless powder. Yield: 66.6 %

M.p. 103 - 105°C (decomp.) IR 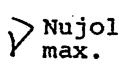 (cm⁻¹) : 1740, 1₇₃₀, ₁₅₉₅, ₁₅₈₅

### Example 46

5.8 g of ethyl 2-n-nonyl-3-ethylaminopropionate, 5.4 g of benzyl (3S)-1,2,3,4-tetrahydroisoquinoline-3-carboxylate and 2.6 g of phosgene and treated in the same manner as described in Example 1. Benzyl (3S)-2-[N-((2R)-2-ethoxycarbonyl- n-undecanyl)-N-ethylcarbamoyl]-1,2,3,4-tetrahydroisoquinoline-3-carboxylate (α-isomer) and benzyl (3S)-2-[N-((2S)-2-ethoxycarbonyl-n-undecanyl)-N-ethylcarbamoyl]-1,2,3,4-tetrahydro- isoqizinoline-3-carboxylate (B-isomer) are obtained, respectively.

α-isomer: Colorless syrup Yield: 3.8 g (33.3 %) IR 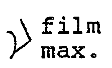 (cm⁻¹) : 1730, ₁₆₅₀

β-isomer: Colorless syrup Yield: 1.8 g (15.8 %) IR 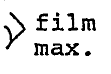 (cm⁻¹) : 1730, 1650

### Example 47

1.8 g of benzyl (3S)-2-[N-((2R)-2-ethoxycarbonyl- n-undecanyl)-N-ethylcarbamoyl]-1,2,3,4-tetrahydroisoquinoline-3-carboxylate are treated in the same manner as described in Example 2-(1). 1.0 g of calcium (3S)-2-[N-((2R)-2-ethoxycarbonyl-n-undecanyl)-N-ethylcarbamoyl]-1,2,3,4-tetrahydroisoquinoline-3-carboxylate is obtained as colorless powder. Yield: 63.6 %

M.p. 82°C (decomp.) IR 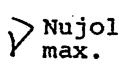 (cm⁻¹) : ₁₇₃₀, ₁₆₄₀, ₁₆₁₀, ₁₅7₀

### Example 48

1.5 g of benzyl (3S)-2-[N-((2R)-2-ethoxycarbonyl-n-undecanyl)-N-ethylcarbamoyl]-1,2,3,4-tetrahydroisoquinoline-3-carboxylate are treated in the same manner as described in Example 6-(1). 0.6 g of (3S)-2-[N-((2R)-2-carboxy-n-undecanyl)-N-ethylcarbamoyl]-1,2,3,4-tetrahydroisoquinoline-3-carboxylic acid is obtained as colorless powder. Yield: 50.6 %

M.p. 104 - 106°C (decomp.) IR 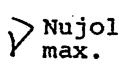 (cm⁻¹) : 1740, 1730, 1595, 1580

### Example 49

(1) 10.0 g of ethyl 2-(2-phenylethyl)-3-ethylamino- propionate are added to 45 ml of an ethyl acetate solution containing 12.0 g of N-benzyloxycarbonyl-D-phenylalanine. 60 ml of isopropylether are added to the solution, and the mixture is allowed to stand at room temperature overnight. Crystalline precipitates are collected by filtration, washed with 15 ml of ethyl acetate-isopropylether (1 : 1) and dried. The precipitates are dissolved in 17 ml of ethyl acetate, and 17 ml of isopropylether are added therto. After the mixture is allowed to stand at room temperature overnight, crystalline precipitates are collected by filtration. ₇.₄ g of ethyl (2R)-2-(2-phenylethyl)-3-ethylaminopropionate _{N}- benzyloxycarbonyl-D-phenylalanine salt are obtained as colorless crystls. Yield: 33.6 %

M.p. 93 - 95 °C 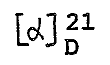 -16.9° (c= 1, ethanol)

50 ml of an aqueous sodium bicarbonate solution are added to 6.0 g of the product, and the mixture is extracted with ether. The extract is washed with an aqeuous sodium chloride solution and dried. Then, ethanolic hydrochloric acid is added thereto, and crystalline precipitates are collected by filtration. 2.8 g of ethyl (2R)-2-(2-phenylethyl)-3-ethylaminopropionate hydrochloride are obtained as colorless crystals. Yield: 89.6 %

M.p. 114 - 116°C (recrystallized from ethanol-ether) 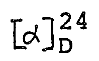 + 18.6° (c= 1, ethanol)

(2) A solution of 7.1 g of ethyl (2R)-2-(2-phenylethyl)-3-ethylaminopropionate and 4.8 g of triethylamine in 20 ml of methylene chloride is added dropwise to 30 ml of a methylene chloride solution containing 3.6 g of phosgene. Said dropwise addition is carried out at about -30°C under stirring. the solution is stirred at the same temperature for 30 minutes and then condensed under reduced pressure. The residue is dissolved in 20 ml of dimethylformamide, and a solution of 7.6 g of benzyl (3S)-1,2,3,4-tetrahydroisoquinoline-3-carboxylate and 4.2 g of triethylamine in 20 ml of dimethylformamide is added dropwise thereto. The mixture is stirred at room temperature overnight. Then, water is added to the reaction mixture, and said mixture is extracted with ether. The extract is washed with diluted hydrochloric acid and an aqueous sodium bicarbonate solution, dried and then evaporated to remove solvent. 12.7 g of benzyl (3S)-2-[N-((2_{R})-2- ethoxycarbonyl-4-phenyl-n-butyl)-N-ethylcarbamoyll-1,2,3,4-tetrahydroisoquinoline-3-carboxylate are obtained as colorless syrup. Yield: 82.3 %

IR 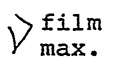 (cm⁻¹) : 1730, 165₀ Mass m/e : 542 (M⁺)

### Example 50

6.0 g of benzyl (3S)-2-[N-((2R)-2-ethoxycarbonyl-4-phenyl-n-butyl)-N-ethylcarbamoylJ-1,2,3,4-tetrahydroiso- guinoline-3-carboxylate obtained in Example 49 are dissolved in 60 ml of ethanol, and 0.2 g of 10 % palladium-carbon is added thereto. The mixture is shaken at room temperature in hydrogen gas atmosphere for 3 hours under an atmospheric pressure. The catalyst is removed by filtration, and the filtrate is condensed to dryness. 4.8 g of (3S)-2-[N-((2R)-2-ethoxycarbonyl-4-phenyl-n-butyl)-N-ethylcarbamoyl]-1,2,3,4-tetrahydroisoquinoline-3-carboxylic acid are obtained as colorless syrup. Yield: 96.0 % The physico-chemical properties of the product are identical with those of the sample obtained in Example 5.

### Example 51

(1) 7.2 g of N-benzyloxycarbonyl-L-phenylalanine, 6.0 g of ethyl 2-(2-phenylethyl)-3-ethylaminopropionate, 20 ml of ethyl acetate and 45 ml of isopropylether are treated in the same manner as described in Example 49-(1). 3.9 g of ethyl (2S)-2-(2-phenylethyl)-3-ethylaminopropionate-N-benzyloxycarbonyl-L-phenylalanine salt are obtained as colorless crystals. Yield: 29.5 %

M.p. 92 - 95°C 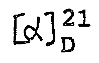 + 16.6° (c= 1, ethanol)

2.5 g of the product are treated in the same manner as described in the second paragraph of Example 49-(1), whereby 1.2 g of ethyl (2S)-2-(2-phenylethyl)-3-ethylamino- propionate hydrochloride are obtained as colorless crystals. Yield: 88.6 %

M.p. 113 - 116°C [α]²⁴_{D} - 18.7° (c= 1, ethanol)

(2) 7.1 g of ethyl (2S)-2-(2-phenylethyl)-3-ethylamino-I propionate, 7.6 g of benzyl (3S)-1,2,3,4-tetrahydroisoquinoline-3-carboxylate, 3.6 g of phosgene, 9 g of triethylamine, 40 ml of methylene chloride and 20 ml of dimethylformamide are treated in the same manner as described in Example 49-(2). 12.3 g of benzyl (3S)-2-[N-((2S)-2-ethoxycarbonyl-4-phenyl-n-butyl)-N-ethylcarbamoyl]-1,2,3,4-tetrahydroisoquinoline-3-carboxylate are obtained as colorless syrup. Yield: 79.7 %

_{IR} film (cm⁻¹) : ₁₇₃₀, 1650 Mass m/e : 542 (M⁺)

### Example 52

6.0 g of benzyl (3S)-2-rN-((2S)-2-ethoxycarbonyl-4-phenyl-n-butyl)-N-ethylcarbamoyl]-1,2,3,4-tetrahydroisoquinoline-3-carboxylate obtained in Example 51 are treated in the same manner as described in Example 50. 4.7 g of (3S)-2-[N-((2S)-2-ethoxycarbonyl-4-phenyl-n-butyl)-N-ethylcarbamoyl]-1,2,3,4-tetrahydroisoquinoline-3-carboxylic acid are obtained as colorless syrup. Yield: 94.0 % Benzylamine salt monohydrate :

Colorless fine crystals (recrystallized from ethyl acetate-isopropylether)

M.p. 95 - 98°C 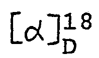 + 5.6° (c= 1, ethanol) IR 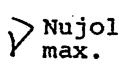 (cm⁻¹) : ₃₅₀₀, ₃₄₅₀, ₁₇₃₅, ₁₆₁₀

### Example 53

(1) 5.1 g of methyl 2-(2-phenylethyl)-3-ethylamino- propionate are added to 20 ml of an ethyl acetate solution containing 6.5 g of N-benzyloxycarbonyl-D-phenylalanine. 10 ml of isopropylether are added to the solution, and the mixture is allowed to stand at room temperature overnight. Crystalline precipitates are collected by filtration, washed with isopropylether, dried and then recrystallized from 25 ml of ethyl acetate. 4.5 g of methyl (2R)-2-(2-phenylethyl)-3-ethylaminopropionate N-benzyloxycarbonyl-D-phenylalanine salt are obtained as colorless crystals. Yield: 38.5 %

M.p. 109 - 111°C 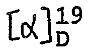 - 6.9 ° (c= 1, methanol)

30 ml of an aqueous potassium carbonate solution are added to 3.0 g of the product, and the mixture is extracted with ether. The extract is washed with an aqeuous sodium chloride solution and dried. Then, ethanolic hydrochloric acid is added thereto, and crystalline precipitates are collected by filtration. 1.45 g of methyl (2R)-2-(2- phenylethyl)-3-ethylaminopropionate hydrochloride are obtained as colorless scales. Yield: 95.1 %

M.p. 142 - 144°C [α]¹⁹_{D} + 23.2° (c= 1, methanol)

(2) 4.7 g of methyl (2R)-2-(2-phenylethyl)-3-ethyl- aminopropionate, 3.8 g of methyl (3S)-1,2,3,4-tetrahydroisoquinoline-3-carboxylate, 2.5 g of phosgene, 6.4 g of triethylamine, 30 ml of methylene chloride and 20 ml of dimethylformamide are treated in the same manner as described in Example 49-(2). 7.2 g of methyl (3S)-2-[N-((2R)-2-methoxycarbonyl-4-phenyl-n-butyl)-N-ethylcarbamoyl]-1,2,3,4-tetrahydroisoquinoline-3-carboxylate are obtained as colorless syrup. Yield: 80.1 %

IR 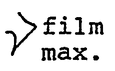 (cm⁻¹) : ₁₇₃₅, ₁₆₅₀ Mass m/e : 452 (M⁺)

### Example 54

4.0 g of methyl (3S)-2-[N-((2R)-2-methoxycarbonyl-4-phenyl-n-butyl)-N-ethylcarbamoyl]-1,2,3,4-tetrahydroisoquinoline-3-carboxylate obtained in Example 53 are dissolved in 60 ml of 90 % aqueous ethanol, and 3.0 g of potassium hydroxide are added thereto. The mixture is stirred at room temperature overnight. After the reaction, the mixture is condensed under reduced pressure to remove solvent, hydrochloric acid is added to the residue, and the aqueous mixture obtained is extracted with ethyl acetate. The extract is washed with an aqueous sodium chloride solution, dried and evaporated to remove solvent. 3.2 g of (3S)-2-[N-((2R)-2-carboxy-4-phenyl-n-butyl)-N-ethylcarbamoyl]-1,2,3,4-tetrahydroisoquinoline-3-carboxylic acid are obtained. Yield: 85.3 %

The physico-chemical properties of the product are identical with those of the sample obtained in Example 6-(1).

### Example 55

(1) A solution of 40.0 g of ethyl 2-(2-phenylethyl)-3-ethylaminopropionate in 150 ml of ether is added to 150 ml of an aqueous solution containing 14 g of potassium carbonate, and 27.5 g of benzyloxycarbonyl chloride are added dropwise thereto at room temperature. The mixture is stirred at the same temperature for 5 hours. After the reaction, the ether layer is separated therefrom, washed with diluted hydrochloric acid, dried and then condensed to dryness, whereby 58.5 g of ethyl 2-(2-phenylethyl)-3-(N-benzyloxycarbonyl-ethylamino)propionate are obtained as colorless syrup. 58.0 g of said propionate are dissolved in 100 ml of ethanol, and a solution of 25 g of sodium hydroxide in 100 ml of water is added dropwise thereto. After the mixture obtained is stirred at room temperature overnight, it is evaporated under reduced pressure to remove ethanol. Then, the aqueous solution thus obtained is washed with ether, made acidic with hydrochloric acid and extracted with ether. The extract is washed with water, dried and condensed. 51.0 g of 2-(2-phenylethyl)-3-(N-benzyloxycarbonyl-ethylamino)-propionic acid are obtained as colorless syrup.

(2) 50.0 g of 2-(2-phenylethyl)-3-(N-benzyloxycarbonyl- ethylamino)propionic acid are dissolved in 400 ml of ethyl acetate,and 23.1 g of L-phenylalanine amide are added thereto. The mixture is heated until L-phenylalanine is dissolved therein. Then, the mixture is condensed to dryness, and 900 ml of ether are added to the residue. After the ethereal mixture is stirred for about one hour, said mixture is allowed to stand at room temperature overnight. Crystalline precipitates are collected by filtration and recrystallized from a mixture of 100 ml of ethyl acetate and 600 ml of ether. 31.0 g of (2R)-2-(2-phenylethyl)-3-(N-benzyloxycarbonyl-ethylamino)propionic acid L-phenylalanine amide salt are obtained as colorless crystals. Yield: 42.4 %

M.p. 99 - 101°C 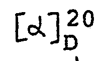 -5.2° (c= 0.5, methanol)

IR 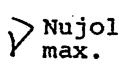 (cm⁻¹) : 33₅₀, 3110, 1700, ₁685, 1630 30.0 g of the product obtained above are suspended in ethyl acetate, 5 % hydrochloric acid is added thereto to make the suspension acidic. Then, the ethyl acetate layer is collected therefrom, washed with water, dried and condensed under reduced pressure. The residue obtained is dissolved in 300 ml of isopropyl ether. 10.4 g of dicyclohexylamine -are added to the solution, and crystalline precipitates are collected by filtration. 28.6 g of (2R)-2-(2-phenylethyl)-3-(N-benzyloxycarbonyl-ethylamino)propionic acid dicyclohexylamine salt are obtained. Yield: 92.3 %

M.p. 102 - 104°C [α]²⁰_{D} - 14.2° (_{C}= 0.66, methanol) IR ν^{Nujol}ₘₐₓ. (cm⁻¹) : ₁₇₀₀, ₁₆₂₀

(3) 15.0 g of (2R)-2-(2-phenylethyl)-3-(N-benzyloxycarbonyl-ethylamino)propionic acid dicyclohexylamine salt are suspended in 50 ml of dimethylformamide, and 8.0 g of ethyl iodide are added thereto. The mixture is stirred at 50°C for 3 hours and then at room temperature overnight. The reaction mixture is condensed under reduced pressure. Ether is added to the residue, and insoluble materials are removed by filtration. The filtrate is washed with an aqueous sodium bicarbonate solution and an aqueous sodium thiosulfate solution, dried and then evaporated to remove solvent. 10.0 g of ethyl (2R)-2-(2-phenylethyl)-3-(N-benzyloxycarbonyl-ethylamino)propionate are obtained as colorless oil. Yield: 93.3 %

10.0 g of the product obtained above are dissolved in 100 ml of ethanol, and 0.2 g of 10 % palladium-carbon is added thereto. The mixture is stirred at room temperature in hydrogen gas atmosphere under an atomospheric pressure until hydrogen uptake is completed. After the reaction, the catalyst is removed by filtration, and the filtrate is evaporated under reduced pressure to remove solvent. 6.2 g of ethyl (2R)-2-(2-phenylethyl)-3-ethylaminopropionate are obtained as colorless oil. Yield: 95.4 %

IR 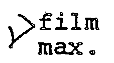 (cm⁻¹) : 1₇30 Mass m/e : 249 (M⁺)

(4) Ethyl (2R)-2-(2-phenylethyl)-3-ethylaminopropionate obtained above is treated in the same manner as described in Example 49-(2), whereby benzyl (3S)-2-IN-((2R)-2-ethoxycarbonyl-4-phenyl-n-butyl)-N-ethylcarbamoyl]-1,2,3,4-tetrahydroisoquinoline-3-carboxylate is obtained.

### Example 56

(1) 5.2 g of ethyl 2-n-octyl-3-ethylaminopropionate, 2.0 g of potassium carbonate, 3.5 g of benzyloxycarbonyl chloride and 1.2 g of sodium hydroxide are treated in the same manner as described in Example 55-(1). 6.3 g of 2-n-octyl-3-(N-benzyloxycarbonyl-ethylamino)propionate are obtained as colorless syrup. Yield: 93.0 %

(2) 4.4 g of 2-n-octyl-3-(N-benzyloxycarbonyl- ethylamino)propionic acid and 2.0 g of L-phenylalanine amide are dissolved in 4 ml of ethyl acetate under heating. 20 ml of n-hexane are added to the solution, and the mixture is allowed to stand at room temperature overnight. Crystalline precipitates are collected by filtration and recrystallized from 32 ml of isopropyl ether. 2.8 g of (2R)-2-n-octyl-3-(_{N}-benzyloxycarbonyl-ethylamino)propionic acid·L-phenylalanine amide salt are obtained as colorless crystals. Yeild: 43.8 %

M.p. 81 - 82°C 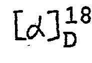 - 13.8° (c= 1, methanol)

2.4 g of the product obtained above are suspended in ether, and 5 % hydrochloric acid is added thereto to make the suspension acidic. The ether layer is collected therefrom, washed with an aqueous sodium chloride solution, and dried. .0.82 g of dicyclohexylamine is added to the ethereal solution, and said solution is condensed. Then, the residue obtained is washed with n-hexane. 2.2 g of (2R)-2-n-octyl-3-(N- benzyloxycarbonyl-ethylamino)propionic acid dicyclohexylamine salt are obtained as colorless crystals. Yield: 88.8 % M.p. 87 - 88°C 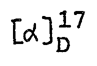 + 18.5° (c= 0.8, methanol)

(3) 2.0 g of (2R)-2-n-octyl-3-(N-benzyloxycarbonyl- ethylamino)propionic acid·dicyclohexylamine salt are treated in the same manner as described in Example 55-(3), whereby 0.75 g of ethyl (2R)-2-n-octyl-3-ethylaminopropionate are obtained as colorless oil. Yield: 86.0 % IR 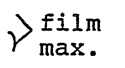 (cm⁻¹) : 1730 Mass m/e : 257 (M⁺)

(4) 2.6 g of ethyl (2R)-2-n-octyl-3-ethylamino- propionate, and 2.7 g of benzyl (3S)-1,2,3,4-tetrahydro- : isoquinoline-3-carboxylate and 1.3 g of phosgene are treated in the same manner as described in Example 49-(2). 4.4 g of benzyl (3S)-2-[N-((2R)-2-ethoxycarbonyl-n-decyl)-N-ethylcarbamoyl]-1,2,3,4-tetrahydroisoquinoline-3-carboxylate are obtained as colorless syrup. Yield: 79.9 %

### Preparation of starting compounds:

[I] To a solution of 22..3 g of potassium hydroxide in 400 ml of ethanol are added 92.0 g of diethyl n-octyl- malonate, and the solution is stirred at room temperature for 3 hours. After the reaction, the solution is evaporated under reduced pressure to remove ethanol. The residue is dissolved in 300 ml of water, and the aqueous solution is washed with ether, made acidic with hydrochloric acid and extracted with ethyl acetate. The extract is dried and evaporated under reduced pressure to remove solvent. The residue obtained is cooled to 0° to -5°C, and 26.6 g of diethylamine are added dropwise thereto. The mixture is stirred for about 3 hours. Then, 29.2 g of 35 % formaline are added thereto and said mixture is further stirred overnight. The reaction mixture is extracted with ether, and the extract is dried and condensed to remove solvent. Then, the residue thus obtained is distilled under reduced pressure. 54.8 g of ethyl 2-n-octylacrylate are obtained as colorless oil. Yield: 76.4 % B.p. 95 - 97°_{C/3} mmHg

The compounds shown in the following Table 1 are obtained in the same manner as described above.

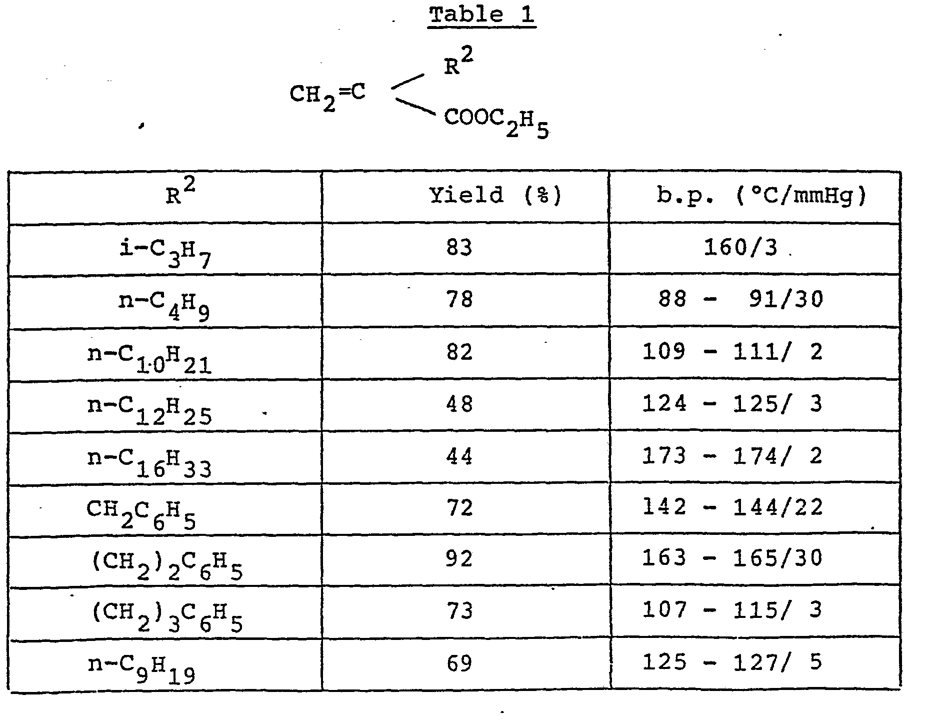

[II] 6.4 g of ethyl 2-n-octylacrylate are dissolved in 10 ml of tetrahydrofuran, and 8.0 g of ethylamine are added thereto. The solution is stirred at room temperature for 2 days. The reaction solution is condensed under reduced pressure, and the residue is distilled under reduced pressure. 5.8 g of ethyl 2-n-octyl-3-ethylaminopropionate are obtained as colorless oil. Yield: 74.7 % B.p. 146 - 150°C/7 mmHg

### Oxalate: M.p. 186 - 187°C

The compounds shown in the following Table 2 are obtained in the same manner as described above.

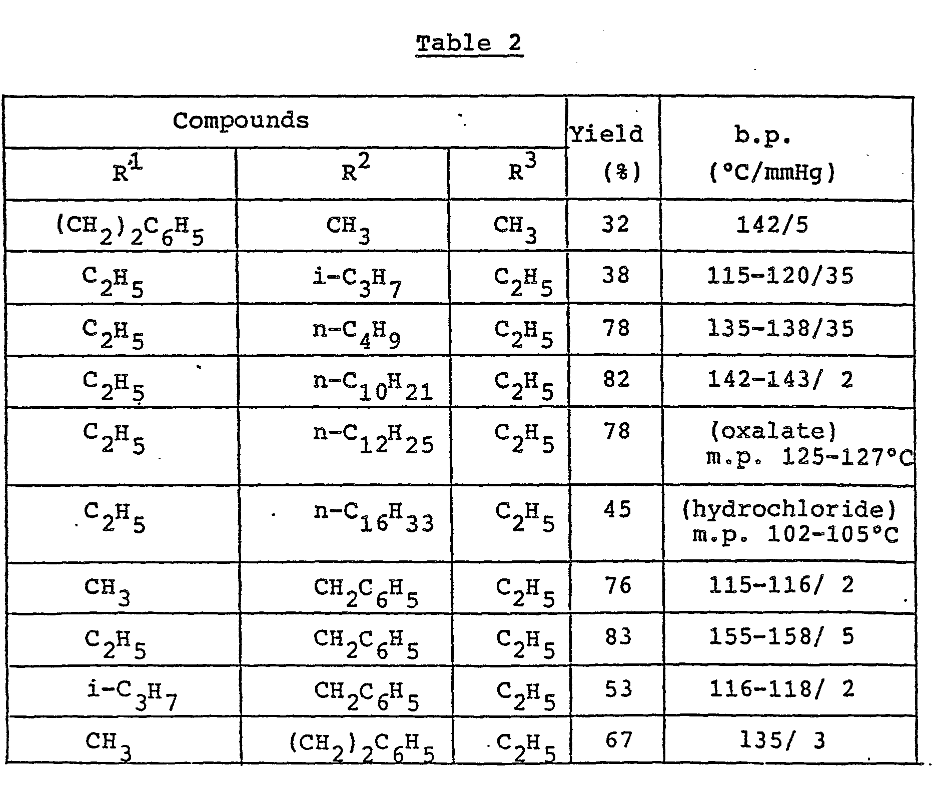

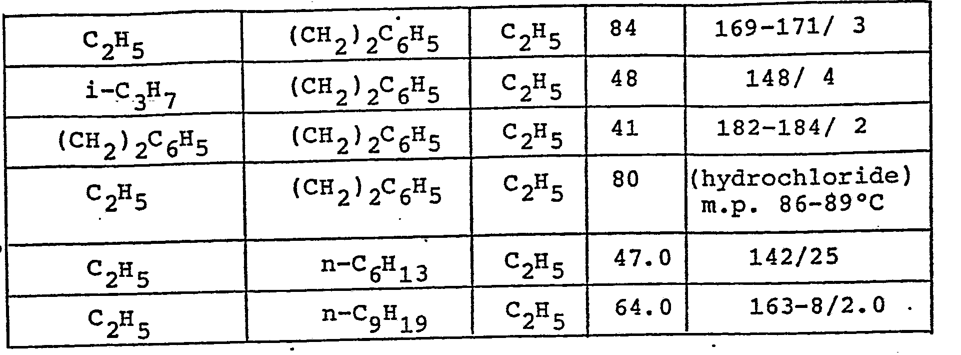

## Claims

1. A compound of the formula: 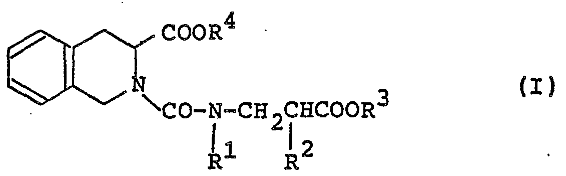 wherein R¹ is lower alkyl or phenyl-lower alky, R² is alkyl or phenyl-lower alkyl, R³ is hydrogen or lower alkyl and _{R}⁴ is hydrogen, lower alkyl or aralkyl.

2. A compound of the formula: 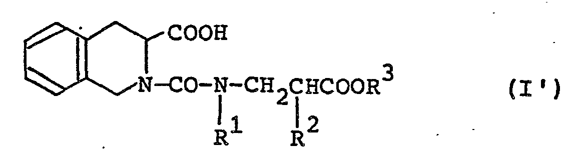 wherein R¹ is lower alkyl or phenyl-lower alkyl, _{R}² is alkyl or phenyl-lower alkyl and R³ is hydrogen or lower alkyl, or a pharmaceutically acceptable salt thereof.

3. The compound claimed in Claim 2 in which _{R}¹ is alkyl of one to 3 carbon atoms or phenethyl, R² is alkyl of one to 16 carbon atoms, benzyl, phenethyl or phenylpropyl, and _{R}³ is hydrogen or alkyl of one to 2 carbon atoms.

4. The compound claimed in Claim 2, in which R¹ is ethyl, R² is alkyl of 6 to 12 carbon atoms or phenethyl, and _{R}³ is hydrogen or ethyl.

5. The compound claimed in Claim 2 in which R¹ is ethyl, R² is alkyl or 8 to 11 carbon atoms and R³ is ethyl.

6. The compound claimed in Claim 2 in which _{R}¹ is ethyl, R² is n-octyl, n-dodecyl or phenethyl and R³ is ethyl.

7. The compound claimed in Claim 2 in which R¹ is ethyl, _{R}² is n-octyl or phenethyl and R³ is ethyl.

8. The compound claimed in either one of Claims 2 through 7 in which the carbon atom at the 3rd-position of isoquinoline ring has S-configuration.

9. The compound claimed in either one of Claims 2 through 7 in which the carbon atom at the 3rd-position of isoquinoline ring has S-configuration and the carbon atom at the 2nd-position of the propionic moiety of the formula: -CH₂CH(R²)COOR³ has R-configuration.

10. The compound claimed in Claim 2 which is (3S)-2-[N-((2R)-2-ethoxycarbonyl-n-decyl)-N-ethylcarbamoyl]-1,2,3,4-tetrahydroisoquinoline-3-carboxylic acid or a pharmaceutically acceptable salt thereof.

11. The compound claimed in Claim 2 which is (3S)-2-[N-((2R)-2-ethoxycarbonyl-4-phenyl-n-butyl)-N-ethylcarbamoyl]-1,2,3,4-tetrahydroisoquinoline-3-carboxylic acid or pharma- cutically acceptable salt thereof.

12. A.process for preparing a compound of the formula: . 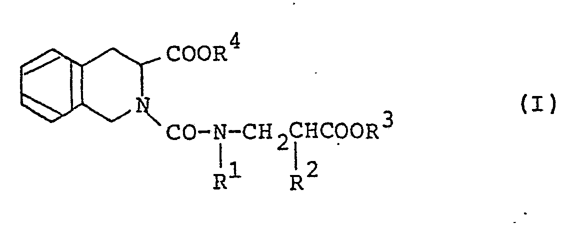 wherein R¹ is lower alkyl or phenyl-lower alkyl, _{R}² is alkyl or phenyl-lower alkyl, R³ is hydrogen or lower alkyl and _{R}⁴ is hydrogen, lower alkyl or aralkyl, which comprises the steps of : reacting a compound of the formula: 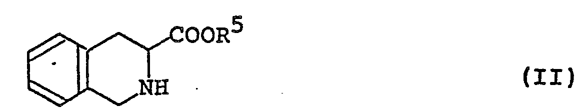 wherein _{R}⁵ is lower alkyl or aralkyl, with phosgene to give a compound of the formula: 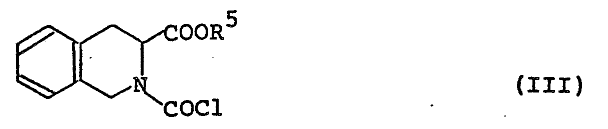 wherein _{R}⁵ is the same as defined above, condensing said compound (III) with an optically active or . racemic compound of the formula: 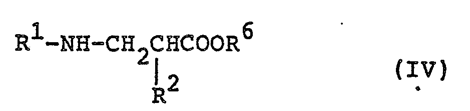 wherein R⁶ is lower alkyl and R¹ and R² are the same as defined above, to give a compound of the formula: 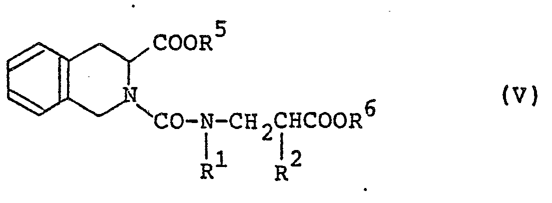 wherein R¹, _{R}², R⁵ and R⁶ are the same as defined above, and if required, further subjecting the compound (_{V}) to cataltic hydrogenation and/or hydrolysis.

13. The process according to Claim 12, wherein the compound (I) in which R³ and/or R⁴ are hydrogen is further converted to a pharmaceutically acceptable salt thereof.

14. A process for preparing a compound of the formula: 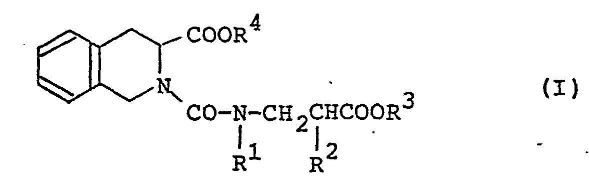 wherein R¹ is lower alkyl or phenyl-lower alkyl, R² is alkyl or phenyl-lower alkyl, R³ is hydrogen or lower alkyl and _{R}⁴ is hydrogen, lower alkyl or aralkyl, which comprises the steps of: reacting an optically active or racemic compound of the formula: 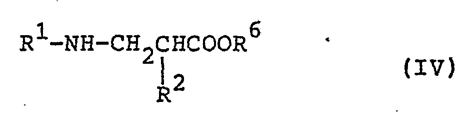 wherein R⁶ is lower alkyl and _{R}¹ and _{R}² are the same as defined above, to give a compound of the formula: 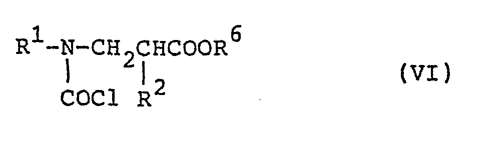 wherein R¹, R² and _{R}⁶ are the same as defined above,
condensing the compound (IV) with a compound of the formula: 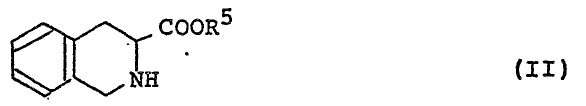 wherein _{R}⁵ is lower alkyl or aralkyl, to give a compound of the formula: 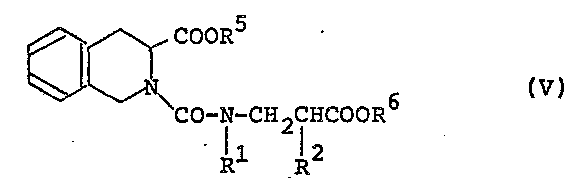 wherein R¹, _{R}², R⁵ and R⁶ are the same as defined above, and
if required, further subjecting the compound (V) to catalytic hydrogenation and/or hydrolysis.

15. The process.according to Claim 14, wherein the compound (I) in which R³ and R⁴ are hydrogen is further converted to a pharmaceutically acceptable salt thereof.

16. A pharmaceutical composition which comprises a therapeutically effective amount of a compound of the formula: 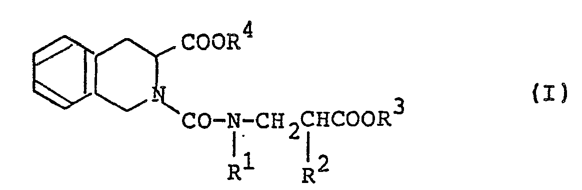 wherein R¹ is lower alkyl or phenyl-lower alkyl, R² is alkyl or phenyl-lower alkyl, R³ is hydrogen or lower alkyl and R⁴ is hydrogen, lower alkyl or aralkyl, and a pharmaceutically acceptable carrier therefor.

17. A pharmaceutical composition which comprises a therapeutically effective amount of a compound of the formula: 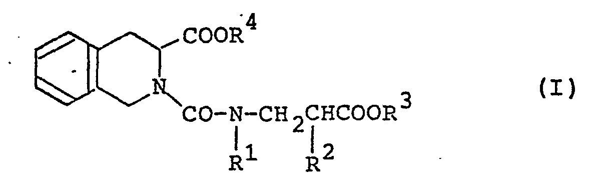 wherein R1 is lower alkyl or phenyl-lower alkyl, _{R}² is alkyl or phenyl-lower alkyl, R³ is hydrogen or lower alkyl and _{R}⁴ is hydrogen, or a pharmaceutically acceptable salt thereof and a pharmaceutically acceptable carrier therefor.
